# EUROPÄISCHE PATENTSCHRIFT

(11) **EP 0 427 765 B1**
(45) Veröffentlichungstag und Bekanntmachung des Hinweises auf die Patenterteilung: **05.01.2000**
(21) Anmeldenummer: 89908987.4
(22) Anmeldetag: 04.08.1989
(51) Int. Cl.: C07D 213/69, A61K 31/44, C07D 401/06, C07D 401/14, A61K 31/495

(54) **ALKOXY-4(1H)-PYRIDON-DERIVATIVE, VERFAHREN ZU DEREN HERSTELLUNG UND DEREN VERWENDUNG ALS ARZNEIMITTEL**
ALKOXY-4(1H)-PYRIDONE DERIVATIVES, PROCESSES FOR PREPARING THEM AND THEIR USE AS DRUGS
DERIVES D'ALKOXY-4(1H)-PYRIDONE, LEURS PROCEDES DE PREPARATION ET LEUR EMPLOI COMME MEDICAMENTS

(30) Priorität: 06.08.1988 DE 3826846
(43) Veröffentlichungstag der Anmeldung: 22.05.1991
(73) Patentinhaber: GÖDECKE AKTIENGESELLSCHAFT, D-10587 Berlin (DE)
(72) Erfinder: ARANDA, Julian, D-7801 Vörstetten (DE); HARTENSTEIN, Johannes, D-7801 Stegen-Wittental (DE); RECK, Reinhard, D-7831 Sexau (DE); SCHÄCHTELE, Christoph, D-7800 Freiburg (DE); RUDOLPH, Claus, D-7800 Freiburg (DE); OSSWALD, Hartmut, D-7830 Emmendingen (DE); WEINHEIMER, Günter, D-7819 Denzlingen (DE)
(74) Vertreter: Mansmann, Ivo
(86) Internationale Anmeldenummer: EP8900927
(87) Internationale Veröffentlichungsnummer: WO9001477

(56) Entgegenhaltungen:
- EP-A- 0 120 670
- US-A- 2 965 641
- Bulletin of the Chemical Society of Japan, Band 52, Nr. 1, Januar 1979 (JP) K. IMAFUKU et al.: "Structure of 5-Hydroxy-2-Hydroxymethyl-4-Pyridones", Seiten 107-110, In der Anmeldung erwähnt
- Journal of Heterocyclic Chemistry, Band 23, Nr. 5, Januar/Februar 1986 Heterocorporation siehe Verbindungen 2-8, In der Anmeldung erwähnt

## Beschreibung

Die Erfindung betrifft neue Alkoxy-4(1H)-pyridon-Derivate der allgemeinen Formel I 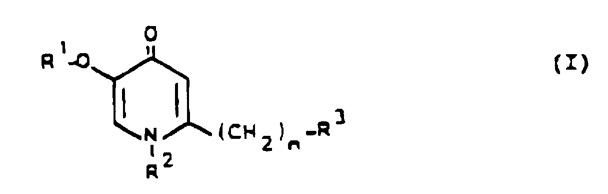 in welcher
- R¹: eine geradkettige oder verzweigte, gesättigte oder ungesättigte Alkyl- oder Adamantylalkylgruppe mit jeweils bis zu 22 Kohlenstoffatomen darstellt,
- R²: eine geradkettige oder verzweigte, gesättigte oder ungesättigte Alkylgruppe mit bis zu 5 Kohlenstoffatomen, eine Cycloalkylmethylgruppe mit 5 bis 7 C-Atomen im Cycloalkylring, eine unsubstituierte oder durch Halogen, Hydroxy, Alkyl mit bis zu 5 C-Atomen, Alkoxy mit bis zu 5 C-Atomen, Dialkylamino mit bis zu 5 C-Atomen oder Benzyloxy substituierte Phenyl- oder phenylalkylgruppe mit einem geradkettigen oder verzweigten Alkylrest mit bis zu 5 C-Atomen oder eine Aminoalkylgruppe der allgemeinen Formel II darstellt 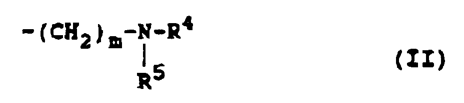 worin R⁴ und R⁵ gleich oder verschieden sind und Wasserstoff oder eine Alkylgruppe mit 1 bis 3 C-Atomen und m eine ganze Zahl von 2 bis 5 bedeuten,
- n: eine ganze Zahl von 1 bis 5 bedeutet, und
- R³: entweder
a) ein Halogenatom, eine Hydroxy-, Cyano-, Carboxamid- oder Alkoxycarbonylgruppe mit bis zu 5 C-Atomen in der Alkylgruppe bedeutet, oder einen Aminorest der allgemeinen Formel III 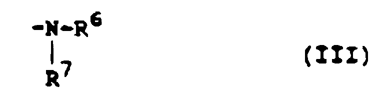 bedeutet, worin R⁶ und R⁷ gleich oder verschieden sind und Wasserstoff, eine Alkyl- oder omega-Hydroxyalkylgruppe mit bis zu 5 C-Atomen, eine unsubstituierte oder durch Halogen, Hydroxy, Alkyl mit bis zu 5 C-Atomen, Alkoxy mit bis zu 5 C-Atomen, Dialkylamino mit bis zu 5 C-Atomen oder Benzyloxy substituierte Phenyl- oder Phenylalkylgruppe mit einem geradkettigen oder verzweigten Alkylrest mit bis zu 5 C-Atomen bedeuten, oder
b) einen Aminorest der allgemeinen Formel IV 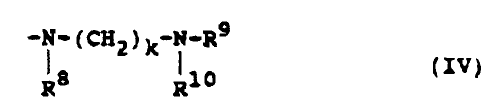 darstellt, in welcher R⁸ Wasserstoff oder eine Alkylgruppe mit bis zu 5 C-Atomen und R⁹ und R¹⁰ gleich oder verschieden sind und Wasserstoff, eine Alkylgruppe mit bis zu 5 C-Atomen, eine unsubstituierte oder durch Halogen, Hydroxy, Alkyl mit bis zu 5 C-Atomen, Alkoxy mit bis zu 5 C-Atomen, Dialkylamino mit bis zu 5 C-Atomen oder Benzyloxy substituierte Phenyl- oder Phenylalkylgruppe mit einem geradkettigen oder verzweigten Alkylrest mit bis zu 5 C-Atomen bedeuten und k eine ganze Zahl von 2 bis 5 bedeutet, oder
c) einen Heterocyclus der allgemeinen Formel V 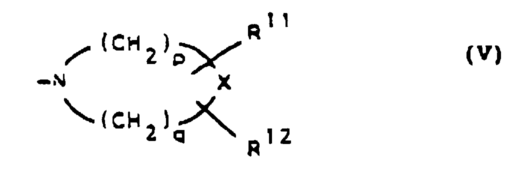 bedeutet, in velcher R¹¹ und R¹² gleich oder verschieden sind und Wasserstoff, eine Alkylgruppe mit bis zu 3 C-Atomen oder eine Phenyl- oder Phenylalkylgruppe mit einem geradkettigen oder verzweigten Alkylrest mit bis zu 5 C-Atomen bedeuten, X für Sauerstoff, einen Aminorest der allgemeinen Formel VI, 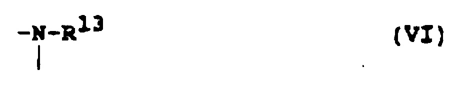 wobei R¹³ Wasserstoff oder eine Phenyl-, Phenylalkyl- oder Diphenylalkylgruppe mit einem geradkettigen oder verzweigten Alkylrest mit bis zu 5 C-Atomen, wobei im Phenylring bis zu 3 CH-Gruppen durch Stickstoffatome ersetzt sein können, bedeutet, oder für einen Rest der allgemeinen Formel VII 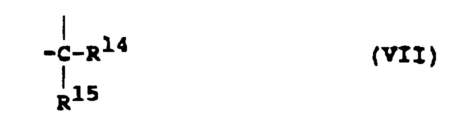 steht, worin R¹⁴ Wasserstoff oder eine unsubstituierte oder durch Halogen, Hydroxy, Alkyl mit bis zu 5 C-Atomen, Alkoxy mit bis zu 5 C-Atomen, Dialkylamino mit bis zu 5 C-Atomen oder Benzyloxy substituierte Phenylgruppe bedeutet, R¹⁵ Wasserstoff, eine Hydroxy-, Hydroxymethyl-, Cyano-, Aminomethyl-, Carboxamid-, Ethoxy- oder Methoxycarbonylgruppe und p und q gleich oder verschieden sein können und 2 oder 3 bedeuten, oder
d) für einen Rest der allgemeinen Formel VIII 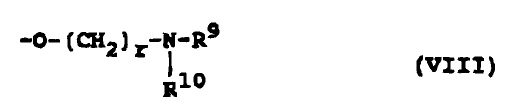 steht, worin R⁹ und R¹⁰ die oben genannte Bedeutung haben, und r eine ganze Zahl von 2 bis 5 bedeutet,
sowie gegebenenfalls deren pharmakologisch unbedenkliche Salze, mit Ausnahme derjenigen Verbindungen, bei denen R¹ einen Alkylrest von 1-6 Kohlenstoffatomen bedeutet.

In der US-A-2 965 641 sind 5-Alkoxy-4(1H)-pyridonederivate beschrieben mit Wirkungen auf Herz und Zentralnervensystem, bei denen R1 eine Alkylgruppe mit 1 bis 6 C-Atomen sein kann. Derartige kurzkettig substituierte verbindungen zeigen jedoch keinen oder einen nur äußerst geringen inhibierenden Effekt auf die Proteinkinase C.

Bevorzugt werden Alkoxy-4(1H)-pyridon-Derivate der allgemeinen Formel I, in welcher
- R¹: eine geradkettige oder verzweigte, gesättigte oder ungesättigte Alkylgruppe mit 10 bis zu 22 Kohlenstoffatomen oder eine Adamantylethylgruppe, und
- R²: einen Methyl-, Ethyl-, Propyl-, Cyclohexylmethylrest, einen unsubstituierten oder durch Halogen, Hydroxy, Methyl, Benzyloxy, Methoxy oder Dimethylamino monosubstituierten Phenyl-, Benzyl- oder Phenylethylrest oder eine Aminoalkylgruppe der allgemeinen Formel II, worin R⁴ und R⁵ eine Methylgruppe und m 2 bis 4 bedeuten,
- n: eine ganze Zahl von 1 bis 3 bedeutet, und
- R³: entweder,
a) wenn n gleich 1 ist, einen Rest der allgemeinen Formel III darstellt, in welcher R⁶ und R⁷ gleich oder verschieden sind und Wasserstoff, eine Methyl-, omega-Hydroxypropyl- oder Benzylgruppe bedeuten, oder
b) einen Aminorest der allgemeinen Formel IV darstellt, in welcher R⁸ Wasserstoff oder eine Methylgruppe bedeutet, R⁹ und R¹⁰ gleich oder verschieden sind und Wasserstoff, eine Methyl-, Benzyl- oder Phenylethylgruppe und k, 2, 3 oder 4 bedeuten, oder
c) einen Heterocyclus der allgemeinen Formel V darstellt, in welcher R¹¹ und R¹² gleich oder verschieden sind und Wasserstoff oder eine Methylgruppe bedeuten, X für einen Aminorest der allgemeinen Formel VI steht, wobei R¹³ Wasserstoff oder eine Phenyl-, Benzyl-, Pyrimidinyl-, Diphenylmethyl- oder Phenylethylgruppe bedeutet, oder X für einen Rest der allgemeinen Formel VII steht, vorin R¹⁴ für eine Phenylgruppe, R¹⁵ eine cyano- oder Aminomethylgruppe bedeutet, und p und q gleich 2 sind.

Insbesondere werden Verbindungen der allgemeinen Formel I, bevorzugt, vorin R³ einen Heterocyclus der folgenden Strukturen Va, Vb, Vc, Vd oder Ve darstellt: 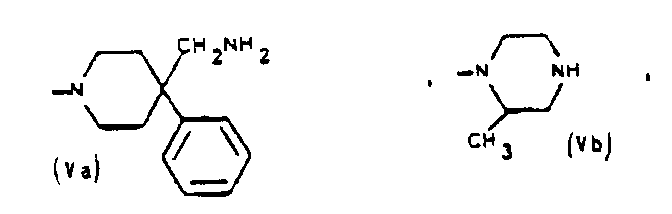 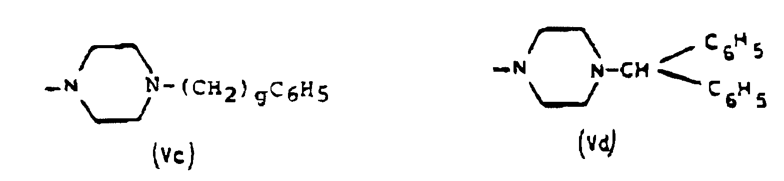 oder 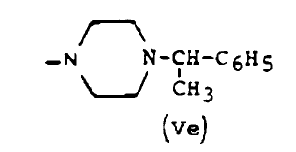 wobei g in Vc 0, 1 oder 2 ist.
Bevorzugt werden auch Verbindungen der allgemeinen Formel I, in welcher R³ entweder, wenn n gleich 1 ist, für einen Rest der allgemeinen Formel VIII steht, worin R⁹ und R¹⁰ Methylgruppen und r 2 oder 3 bedeuten, oder, wenn n gleich 2 oder 3 ist, eine Amino-, Cyano-, Hydroxy-, Methoxycarbonyl- oder Carboxamidgruppe darstellt.

Die Erfindung betrifft auch Verfahren zur Herstellung von Alkoxy-4(1H)-pyridon-Derivaten der allgemeinen Formel I, dadurch gekennzeichnet, daß man entweder:
a) eine Verbindung der allgemeinen Formel IX 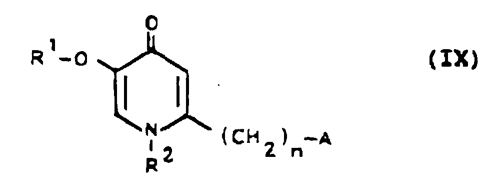 in velcher R¹, R² die oben angegebene Bedeutung haben, n gleich 1 ist und A für eine nukleofuge Abgangsgruppe steht, mit Aminoverbindungen der allgemeinen Formel X, XI und XII, 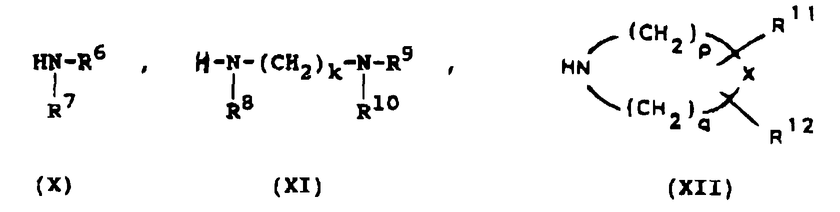 in welcher R⁶, R⁷, R⁸, R⁹, R¹⁰, R¹¹, R¹², k, p, q und X die oben angegebene Bedeutung besitzen, in an sich bekannter Weise umsetzt, oder
b) eine Verbindung der allgemeinen Formel XIII, 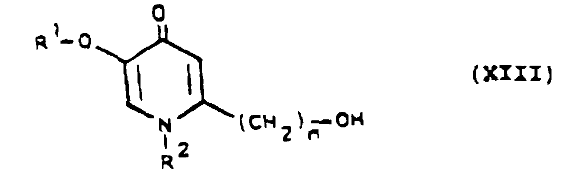 in welcher R¹, R² die oben angegebene Bedeutung haben und n gleich 1 ist, mit einer Verbindung der allgemeinen Formel XIV, 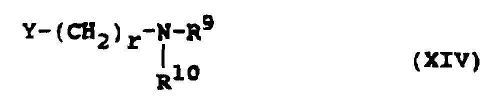 in welcher R⁹, R¹⁰ und r die oben genannte Bedeutung haben und Y eine Ausgangsgruppe bedeutet, in einem geeigneten Lösungsmittel unter basischen Bedingungen, umsetzt, oder
c) wenn n gleich 2 oder 3 ist und R³ für eine Amino-, Hydroxy-, Cyano-, Methoxycarbonyl- oder Carboxamidgruppe steht, ein 2-Halogenmethylpyridon der allgemeinen Formel IXa, 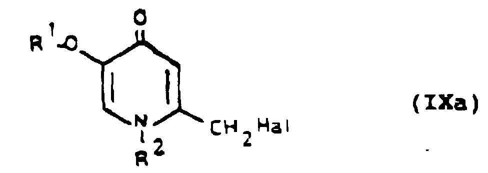 in welcher R¹ und R² die oben angegebene Bedeutung haben, mit einem Cyanessigsäureester nach an sich bekannter Weise umsetzt, verseift und decarboxyliert. Aus den so erhaltenen Cyanoverbindungen der allgemeinen Formel Ia 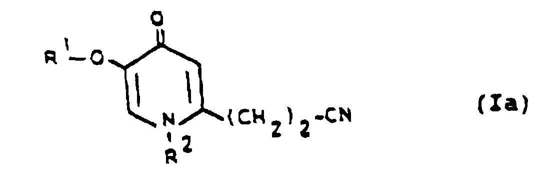 werden dann nach literaturbekannten Methoden die Ester-, Amino-, Hydroxy- und Amidoverbindungen der allgemeinen Formel Ib, Ic, Id und Ie hergestellt. Das folgenden Formelschema veranschaulicht dieses Verfahren.

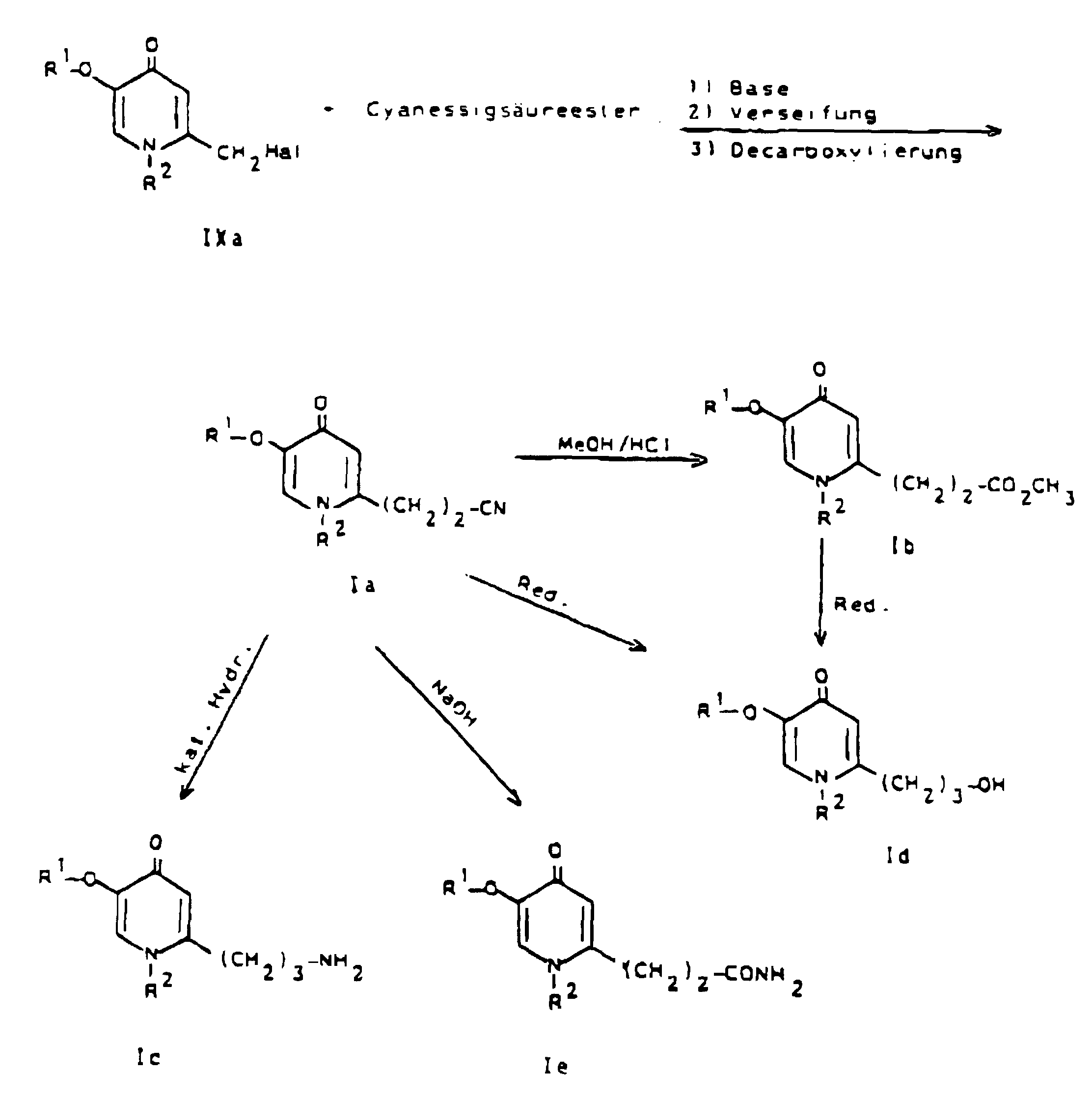

Die Umsetzungen nach verfahren a) werden in Gegenwart eines inerten Lösungsmittels wie Methanol, Ethanol, Propanol oder 2-Propanol, vorzugsweise Ethanol oder 2-Propanol, bei Temperaturen von 20-90°C, bevorzugt bei 60-80°C, durchgeführt. Die Abgangsgruppe A stellt bevorzugt ein Halogenatom, insbesondere Brom dar.

Bei einer zweckmäßigen Ausführungsform zur nukleophilen Substitution des Restes A in IX durch die verwendeten Aminoverbindungen der Formel X, XI und XII vird die Umsetzung in Gegenwart einer Base als säurebindendes Mittel durchgeführt; bevorzugt wird Triethylamin oder Kaliumcarbonat eingesetzt. Gegebenenfalls ist es zweckmäßig, das zur Umsetzung verwendete Aminoderivat in überschüssiger Menge gleichzeitig als säurebindendes Mittel zu verwenden. Die Reaktionszeiten bewegen sich zwischen 1 und 8 Stunden, meist liegen sie bei 2 bis 3 Stunden. Eine Reinigung der erhaltenen Produkte erfolgt in üblicher Weise, beispielsweise durch Umkristallisieren aus einem Lösungsmittel, Überführen in ein Säureadditionssalz oder Säulenchromatographie.

Für den Fall, daß in der allgemeinen Formel I R³ ein Heterocyclus der Formel Va ist, 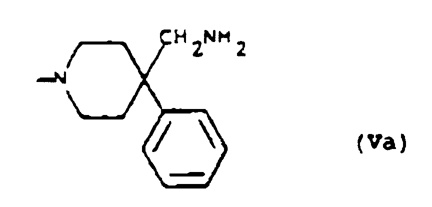 wird zuerst eine Verbindung der allgemeinen Formel IX mit 4-Cyano-4-phenyl-piperidin (XIIa) ungesetzt und danach die Cyanogruppe zum Aminomethyl mit Raney-Nickel und Wasserstoff reduziert. Die Reaktion erfolgt zweckmäßig im Autoklaven mit Methanol, mit Ammoniak gesättigt, bei einer Temperatur zwischen 40-100°C, bevorzugt bei 60°C, und einem Druck von 40-120 bar, bevorzugt bei 60 bar.

Im Fall, daß in der allgemeinen Formel I R³ ein Heterocyclus der Formel Vb ist, 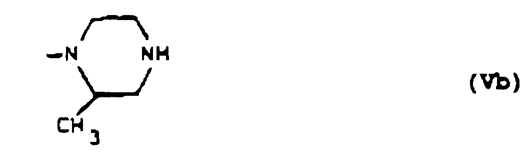 wird eine Verbindung der allgemeinen Formel IX mit einem Piperazinderivat der allgemeinen Formel XIIb, 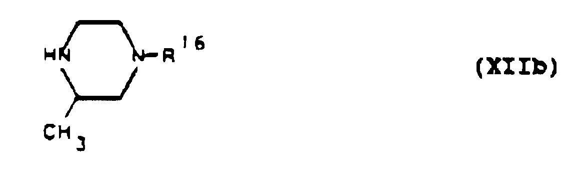 in welcher R¹⁶ eine Schutzgruppe bedeutet, beispielsweise eine Ethoxy-, Methoxy-, tert.-Butoxy- oder Benzyloxycarbonylgruppe, bevorzugt eine Methoxycarbonylgruppe, umgesetzt und anschließend die Schutzgruppe abgespalten. Die Abspaltung der Schutzgruppe geschieht nach an sich üblichen Methoden unter sauren Bedingungen. Vorzugsweise wird die Abspaltung der Schutzgruppe R¹⁶ mit Bromwasserstoff in Eisessig in einem geschlossenen Gefäß unter Normaldruck bei Temperaturen zwischen 5 und 25°C, bevorzugt bei 20°C, durchgeführt.

Bei der Verfahrensvariante b) erfolgt die Durchführung der Reaktion bevorzugt in einem polaren, aprotischen Lösungsmittel wie z.B. Dimethylformamid oder Dimethylsulfoxid, in Gegenwart einer geeigneten Base, vorzugsweise Natriumhydrid (vgl. Eur. Pat. Appl. EP 171,814). Die Umsetzungen werden bei Temperaturen von 20-100°C, zweckmäßig bei Temperaturen von 50-60°C, durchgeführt. Eine Reinigung der erhaltenen Produkte erfolgt wiederum durch Umkristallisieren aus einem Lösungsmittel, Überführen in ein Säureadditionssalz oder Säulenchromatographie.

Die austretende Gruppe Y der Ausgangsverbindung der allgemeinen Formel XIV stellt ein Halogenatom, vorzugsweise Chlor oder Brom, insbesondere Chlor, dar. Weiterhin kommen beispielsweise als Abgangsgruppe aromatische oder aliphatische Sulfonsäurereste in Betracht, wie der p-Toluolsulfonsäure- oder der Methansulfonsäurerest.

Bei der Verfahrensvariante c) werden die Verbindungen der allgemeinen Formel Ia hergestellt, indem man ein 2-Brommethylpyridon-Derivat der allgemeinen Formel IXa, worin R¹ und R² die angegebene Bedeutung haben, mit Cyanessigsäure-tert.-butylester umsetzt. Zweckmäßigerweise wird die Reaktion in einem inerten, polaren aprotischen Lösungsmittel, bevorzugt Dimethylformamid, in Gegenwart einer Base, bevorzugt Kalium-tert.-butylat, bei Temperaturen von 20-100°C, bevorzugt bei 50-60°C, durchgeführt. Die so erhaltenen Zwischenprodukte werden einer sauren oder alkalischen Hydrolyse unterworfen. Vorzugsweise wird alkalisch hydrolysiert, beispielsweise bei Zimmertemperatur in wäßriger Kali- oder Natronlauge und Methanol. In den erhaltenen Verbindungen wird nun die Carbonsäuregruppe nach an sich bekannten Methoden, z.B. durch Schmelzen oder Erhitzen in einem hochsiedenden Lösungsmittel, abgespalten.

Durch katalytische Hydrierung der Cyanogruppe in den Verbindungen der allgemeinen Formel Ia werden die Verbindungen der allgemeinen Formel Ib hergestellt. Die Reaktion erfolgt zweckmäßig im Autoklaven mit methanolischem Ammoniak und Raney-Nickel als Katalysator bei Temperaturen zwischen 40-100°C, bevorzugt bei 60°C und einem Druck von 40-120 bar, bevorzugt bei 60 bar.

Für den Fall, daß bei den Verbindungen der allgemeinen Formel I R³ eine Methoxycarbonylgruppe bedeutet und n gleich 2 ist, läßt man die entsprechenden Cyanoverbindungen der allgemeinen Formel Ia mit methanolischem Chlorwasserstoff reagieren. Vorzugsweise wird die Reaktion in einem geschlossenen Gefäß unter Normaldruck bei Zimmertemperatur und nachfolgender Hydrolyse durchgeführt. Durch Umsetzen einer Verbindung der allgemeinen Formel Ia mit Natriumborhydrid in siedendem 2-Propanol und nachfolgender Behandlung mit Natriumhydroxidlösung werden die Alkoxy-4(1H)-pyridon-Derivate der allgemeinen Formel Id und Ie erhalten (vgl. R.A. Egli, Helv. Chim. Acta 53, 47 (1970)).

Die Verbindungen der allgemeinen Formel Ia-Ie können beispielsweise durch Säulenchromatographie, Überführen in eine Säure und/oder Umkristallisieren aus einem Lösungsmittel gereinigt werden. Gegebenenfalls können die Verbindungen der allgemeinen Formel Ia in an sich bekannter weise, durch Hydrolyse mit wäßrigen oder alkoholischen Alkalimetallhydroxiden, beispielsweise Kaliumhydroxid oder Natriumhydroxid, bei Raumtemperatur in die Verbindungen der allgemeinen Formel Ie umgewandelt werden.

Gegebenenfalls können die Verbindungen der Formel Ib durch Reduktion des Carbonsäureesterrestes in die Verbindungen der allgemeinen Formel Id umgewandelt werden. Die Reduktion kann nach allgemein bekannten Methoden durchgeführt werden, z.B.mit komplexen Methallhydriden wie Natriumborhydrid in tert.-Butanol/Methanol (vgl. K. Soai, Synthetic Communications 12, 463 (1982)).

Die als Zwischenprodukte eingesetzten 4(1H)-Pyridon-Derivate der allgemeinen Formel IX und XIII, in welcher R¹ und R² die angegebene Bedeutung haben, n gleich 1 ist und A für ein Bromatom steht, werden aus Kojisäure nach literaturbekannten Verfahren, wie das folgende Reaktionsschema zeigt, hergestellt:

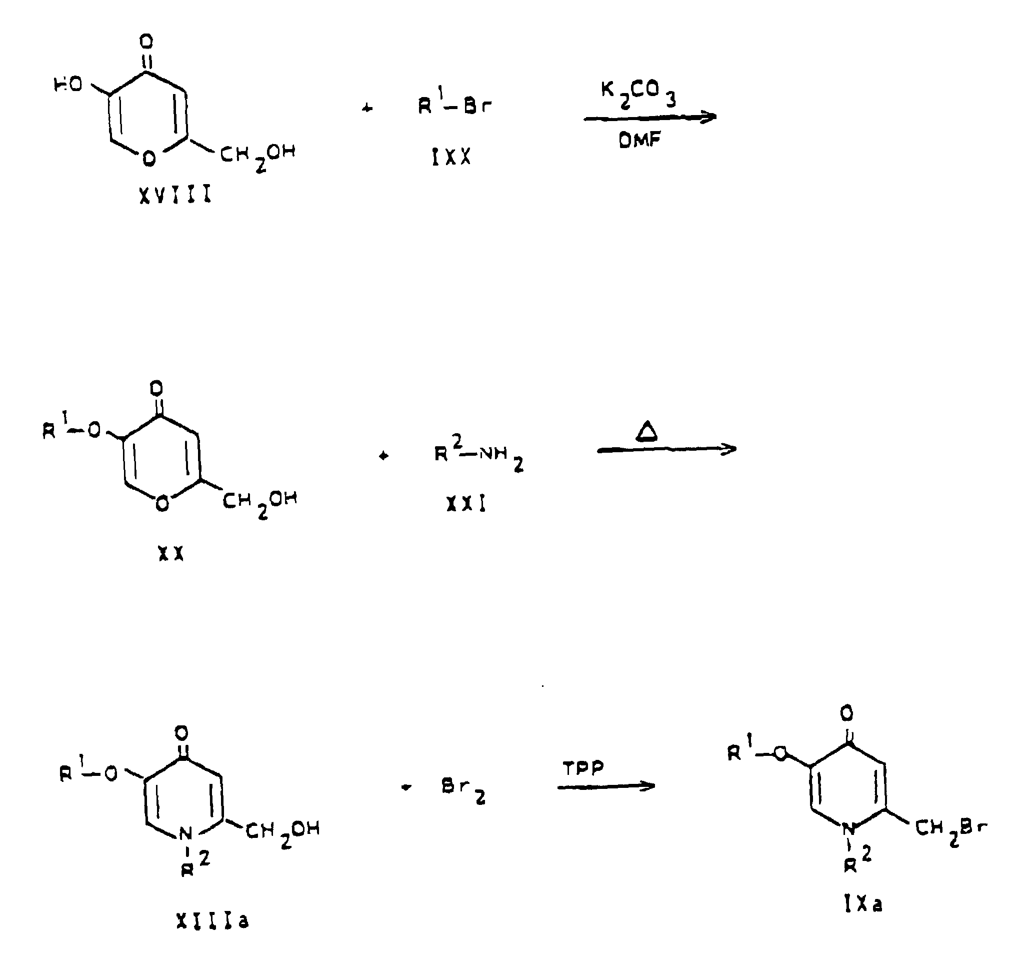

Dabei wird Kojisäure (XVIII) mit dem ensprechenden Alkylbromid IXX nach üblichen, in der Literatur beschriebenen Verfahren (Z.B. US 4644-071-A; Eur. Pat. Appl. EP 171,814; A.F. Thomas und A. Marxer, Helv. Chim. Acta 43, 469 (1960); Eur. Pat. Appl. EP 0209751) eingesetzt. Bevorzugt wird diese Reaktion mit kaliumcarbonat als Base und Dimethylformamid als Lösungsmittel durchgeführt.

Die 2-Hydroxymethyl-4(1H)-pyridon-Derivate der allgemeinen Formel XIIIa werden hergestellt, indem man nach an sich bekannter Weise (vgl. z.B. Counsell et al., J. Med. Chem. 17(1), 1-5 (1974); J.H. Looker and M.D. Cliffton, J. Xeterocyclic Chem. 23, 5 (1986); Tsutomu Teitei, Austr. J. Chem. 36, 2307-15 (1983); Can. 978,958; S. Hünig und G. Köbrich, Liebigs Ann. Chem. 609, 181 (1958): K. Imafuku et al., Bull. chem. Soc. Japan 52, 107 (1979)) ein Pyranon-Derivat der allgemeinen Formel XX mit einem Amin der allgemeinen Formel XXI bei Temperaturen von 80-120°C, bevorzugt bei 100°C, reagieren läßt. Bei Aminen mit niedrigem Siedepunkt wird die Reaktion im Autoklaven in Gegenwart eines inerten, polaren Lösungsmittels, beispielsweise Ethanol, durchgeführt. Die Produkte werden durch Säulenchromatographie oder Kristallisation gereinigt.

Für die Überführung der Hydroxymethylgruppe in Verbindungen der Formel XIII in eine Brommethylgruppe eignet sich besonders das aus Brom und Triphenylphosphin gebildete Triphenylphosphindibromid (vgl. Fieser and Fieser, Reagents for Organic Synthesis, Wiley-Interscience, 1975, Vol. 5, p. 729). Die Durchführung der Reaktion erfolgt, indem man eine Lösung oder Suspension des in situ hergestellten Triphenylphosphindibromids in einem geeigneten, wasserfreien Lösungsmittel, bevorzugt Dichlormethan oder Toluol, mit der betreffenden 2-Hydroxymethyl-4(1H)-pyridon Verbindung der Formel XIII umsetzt, im Temperaturbereich von 30-100°C, vorzugsweise jedoch bei 50°C, wenn Dichlormethan oder bei 70°C, wenn Toluol das Lösungsmittel ist. Die 2-Brommethylverbindungen der Formel IXa werden nach der Reaktion als Hydrobromide isoliert und durch Kristallisation gereinigt oder ohne Reinigung weiter umgesetzt.

Sofern die erfindungsgemäßen Verbindungen der Formel I ein chirales Zentrum aufweisen, können sie entweder als racemische Gemische oder in Form der Enantiomeren vorliegen. Racemische Gemische lassen sich mit den üblichen Methoden in die Enantiomeren trennen.

Da die Verbindungen der allgemeinen Formel I basische Zentren besitzen, überführt man sie zum Zwecke der Reinigung und/oder aus galenischen Grunden mit Hilfe von anorganischen und organischen Säuren bevorzugt in kristalline, pharmakologisch verträgliche Salze. Als Säuren kommen z.B. Salzsäure, Schwefelsäure, Phosphorsäure, Bromwasserstoffsäure, Essigsäure, Weinsäure, Milchsäure, Zitronensäure, Äpfelsäure, Salicylsäure, Ascorbinsäure, Malonsäure, Fumarsäure, oxalsäure oder Bernsteinsäure in Frage. Die Säureadditionssalze werden in der Regel in an sich bekannter Weise durch Mischen der freien Base oder deren Lösungen mit der entsprechenden Säure oder deren Lösungen in einem organischen Lösunsgsmittel, beispielsweise einem niederen Alkohol wie Methanol, Ethanol oder 2-Propanol oder einem niederen Keton wie Aceton oder 2-Butanon oder einem Ether wie Diethylether, Diisopropylether, Tetrahydrofuran oder Dioxan, erhalten.

Die erfindungsgemäßen Verbindungen der allgemeinen Formel I weisen interessante pharmakologische Eigenschaften auf. Insbesondere hemmen sie die Proteinkinase C, ein Calcium- und Phospholipid-abhängiges Schlüsselenzym, das bei der intrazellulären Signalkettentransduktion eine entscheidende Rolle spielt (Y. Nishizuka, Science 233, 305-312 (1986)) und eng mit der Regulation von kontraktilen, sekretorischen und proliferativen Prozessen verknüpft ist.

Aufgrund dieser Eigenschaften können die erfindungsgemäßen Verbindungen zur Prävention und/oder Behandlung von Herz- und Gefäßkrankheiten wie Thrombosen, Arteriosklerose, Hypertonien, von Entzündungsprozessen, Allergien, Krebs und bestimmten degenerativen Schäden des Zentralnervensystems verwendet werden. Ein weiterer Gegenstand der vorliegenden Erfindung ist daher die Verwendung von Alkoxy-4(1H)-pyridon-Derivaten der allgemeinen Formel I bei der Behandlung von Herz- und Gefäßkrankheiten, Entzündungsprozessen, Allergien, Krebs und Erkrankungen des Zentralnervensystems.

Überraschenderweise wurde bei den erfindungsgemäßen Verbindungen außerdem gefunden, daß sie eine endothelabhängige Relaxation der Gefäßmuskulatur bewirken.

Die erfindungsgemäßen Verbindungen der allgemeinen Formel I können in flüssiger oder fester Form oral oder parenteral appliziert werden. Als Injektionslösung kommt vor allem Wasser zur Anwendung, welches die bei Injektionslösungen üblichen Zusätze wie Stabilisierungsmittel, Lösungsvermittler oder Puffer enthält

Derartige Zusätze sind z. B. Tartrat- und Citrat-Puffer, Ethanol, Komplexbildner (wie Äthylendiamin-tetraessigsäure und deren nicht-toxische Salze) sowie hochmolekulare Polymere (wie flüssiges Polyäthylenoxid) zur Viskositätsregulierung, Feste Trägerstoffe sind z. B. Stärke, Lactose, Mannit, Methylcellulose, Talkum, hochdisperse Kieselsäuren, höhermolekulare Fettsäuren (wie Stearinsäure), Gelatine, Agar-Agar, Calciumphosphat Magnesiumstearat, tierische und pflanzliche Fette, feste hochmolekulare Polymere (wie Polyäthylenglykol); für orale Applikation geeignete Zubereitungen können gewünschtenfalls zusätzliche Geschmacks- und/oder Süßstoffe enthalten. Die enteral oder parenteral verabreichten Einzeldosen liegen im Bereich von 0,5 bis 1000 mg, vorzugsweise in Dosen von 1 bis 100 mg.

Die vorliegende Erfindung wird durch die nachfolgenden Ausführungsbeispiele näher erläutert.

### Beispiel 1

### 2-Hydroxymethyl-5-octadecyloxy-4-pyranon

Eine Mischung aus 56,8 g (0,4 Mol) Kojisäure, 55,3 g (0,4 Mol) K₂CO₃, 133,3 g (0,4 Mol) 1-Octadecylbromid, 1 g kaliumjodid und 450 ml absoluten Dimethylformamid wird 10 Stunden bei 90°C gerührt. Nach dem Abkühlen wird die Mischung mit 1,5 Liter Wasser versetzt, der sich gebildete Niederschlag abfiltriert, einmal mit Ethylacetat gut verrührt, abfiltriert und aus 2-Propanol unter Zusatz von Aktivkohle umkristallisiert. Man erhält ein fast farbloses Produkt vom Schmp. 71-73°C.

In analoger weise, wie in Beispiel 1 beschrieben, erhält man:

| Beispiel Nr. | |
|---|---|
| 2 | 2-Hydroxymethyl-5-tetradecyloxy-4-pyranon, Schmp. 65-68°C aus 2-Propanol; |
| 3 | 5-Eicosanyloxy-2-hydroxymethyl-4-pyranon, Schmp. 81-84°C aus 2-Propanol; |

### Beispiel 4 (Herstellungsverfahren für Beispiel 5)

### 5-Butyloxy-2-hydroxymethyl-4-pyranon

Zu einem Gemisch von 50 g (0,352 Mol) Kojisäure, 48,7 g (0,352 Mol) K₂CO₃, 1 g Kaliumjodid und 420 ml absoluten Dimethylformamid werden unter kräftigen Rühren bei Raumtemperatur 48,3 (0,352 Mol) 1-Butylbromid zugetropft. Danach wird die Reaktionsmischung 3 Stunden bei 90°C gerührt. Nach dem Abkühlen wird das Lösungsmittel im Vakuum abgezogen, der Rückstand mit Wasser versetzt und mit Dichlormethan extrahiert.

### Die organische Phase vird abgetrennt, getrocknet, das Lösungsmittel abgedampft und der Rückstand aus Ethylacetat umkristallisiert. Man erhält ein fast farbloses Produkt vom schmp. 68-73°C.

In analoger Weise, wie in Beispiel 4 beschrieben, erhält man:

| Beispiel Nr. | |
|---|---|
| 5 | 5-Decyloxy-2-hydroxymethyl-4-pyranon, Schmp. 46-50°C aus Ethylacetat; |

### Beispiel 6

### 2-Hydroxymethyl-5-(9-cis-octadecenyloxy)-4-pyranon

Eine Mischung aus 14,2 g (0,1 Mol) Kojisäure, 13,8 g (0,1 Mol) K₂CO₃, 1 g Kaliumjodid, 33,14 g (0,1 Mol) 1-Brom-9-cis-octadecen und 120 ml absolutes Dimethylformamid wird 3 Stunden bei 90°C gerührt. Nach dem Abkühlen wird das Lösungsmittel im Vakuum abgezogen, der Rückstand mit Wasser versetzt und mit Dichlormethan extrahiert. Die organische Phase wird abgetrennt, getrocknet und eingedampft. Der Rückstand wird säulenchromatographisch an Kieselgel mit Dichlormethan-Methanol 100:1 gereinigt. Man erhält ein gelbes Öl.
¹H-NMR (90 MHz: CDCl₃), delta (ppm): 0,85 (t, 3H); 1,30 (s, 22H); 1,7-2,2 (m, 6H); 3,8 (t, 2H); 4,2 (t, 1H) ; 4,4 (d, 2H); 5,3 (t, 2H); 6,5 (s, 1H); 7,55 (s, 1H).

In analoger Weise, wie in Beispiel 6 beschrieben, erhält man:

| Beispiel Nr. | |
|---|---|
| 7 | 2-Hydroxymethyl-5-(2-octyldecyloxy)-4-pyranon, gelbes Öl, IR (KBr): 1640 (C=O); |
| 8 | 5-[2-(1-Adamantyl)ethoxy]-2-hydroxymethyl-4-pyranon, Schmp. 155-157°C aus 2-Propanol/Wasser; |

### Beispiel 9

### 1-Benzyl-2-hydroxymethyl-5-octadecyloxy-4(1H)-pyridon

Eine Mischung aus 50,0 g (0,127 Mol) 2-Hydroxymethyl-5-octadecyloxy-4-pyranon und 70 ml Benzylamin wird 2 Stunden bei 100-110°C gerührt. Nach Abkühlen auf ca. 50°C wird die dunkle Lösung mit 400 ml Wasser versetzt und verrührt. Der so gebildete Niederschlag wird abfiltriert, gewaschen, getrocknet und aus 2-Propanol unter Zusatz von Aktivkohle umkristallisiert. Man erhält farblose Kristalle vom Schmp. 120-121°C.

In analoger Weise, wie in Beispiel 9 beschrieben, erhält man:

| Beispiel Nr. | |
|---|---|
| 11 | 1-Benzyl-5-decyloxy-2-hydroxymethyl-4(1H)-pyridon, Schmp. 128-129°C aus 2-Propanol; |
| | |
| 12 | 1-Benzyl-2-hydroxymethyl-5-tetradecyloxy-4(1H)-pyridon, Schmp. 121-123°C aus 2-Propanol; |
| | |
| 13 | 1-Benzyl-5-eicosanyloxy-2-hydroxymethyl-4(1H)-pyridon, Schmp. 120-122°C aus 2-Propanol; |
| | |
| 14 | 1-Benzyl-2-hydroxymethyl-5-(9-cis-octadecenyloxy)-4(1H)-pyridon, Schmp. 92-93°C aus Ethanol; |
| | |
| 15 | 2-Hydroxymethyl-1-(4-methylbenzyl)-5-octadecyloxy-4(1H)-pyridon, Schmp. 122-125°C aus 2-Propanol; |
| | |
| 16 | 1-(4-Chlorbenzyl)-2-hydroxymethyl-5-octadecyloxy-4(1H)-pyridon, Schmp. 125-127°C aus 2-Propanol; |
| | |
| 17 | 2-Hydroxymethyl-1-(4-methoxybenzyl)-5-octadecyloxy-4(1H)-pyridon, Schmp. 120-122°C aus 2-Propanol; |
| | |
| 18 | 2-Hydroxymethyl-1-(2-methoxybenzyl)-5-octadecyloxy-4(1H)-pyridon, Schmp. 107-110°C aus Diisopropylether/2-Propanol; |
| | |
| 19 | 1-Cyclohexylmethyl-2-hydroxymethyl-5-octadecyloxy-4(1H)-pyridon, Schmp. 126-128°C aus 2-Propanol; |
| | |
| 20 | 1-(4-Dimethylaminobenzyl)-2-hydroxymethyl-5-octadecyloxy-4(1H)-pyridon, Schmp. 109-114°C aus Ethanol; |
| | |
| 21 | 1-(3-Dimethylaminopropyl)-2-hydroxymethyl-5-octadecyloxy-4(1H)-pyridon, Schmp. 87-90°C aus Ethylacetat; |
| | |
| 22 | 1-(4-Benzyloxybenzyl)-2-hydroxymethyl-5-octadecyloxy-4(1H)-pyridon, Schmp. 116-120°C, mit Ether verrührt; |
| | |
| 23 | 1-Benzyl-2-hydroxymethyl-5-(2-octyldecyloxy)-4(1H)-pyridon, gelbes Öl, IR (KBr): 1630 (C=O); |
| | |
| 24 | 5-[2-(1-Adamantyl)ethoxy]-1-benzyl-2-hydroxymethyl-4(1H)-pyridon, Schmp. 246-248°C, mit Ether verrührt; |

### Beispiel 25

### (±)-2-Hydroxymethyl-5-octadecyloxy-1-(1-phenylethyl)-4(1H)-pyridon

Eine Mischung aus 8,0 g (0,02 Mol) 2-Hydroxymethyl-5-octadecyloxy-4-pyranon, 4,8 g (0,04 Mol) (±)-1-Phenylethylamin, 1,5 g Na₂CO₃, 10 ml Wasser und 100 ml Ethanol vird 72 Stunden unter Rückfluß gekocht. Nach dem Abkühlen vird das Lösungsmittel und überschüssiges Amin im Vakuum abdestilliert, der Rückstand mit Wasser versetzt und mit Dichlormethan extrahiert. Der Extrakt wird über Na₃SO₄ getrocknet, eingedampft und das erhaltene Harz zur Reinigung an Kieselgel mit Dichlormethan mit 1-2% Methanol chromatographiert. Man erhält ein gelbes Öl, welches beim Stehen wachsartig wird. IR (KBr): 1630 (C=O), MS (m/l) 497 (M⁺).

In analoger Weise, wie in Beispiel 25 beschrieben, erhält man:

| Beispiel Nr. | |
|---|---|
| 26 | (-)-2-Hydroxymethyl-5-octadecyloxy-1-(1-phenylethyl)-4(1H)-pyridon, wachsartig, IR (KBr): 1630 (C=O), [α]_{D} = -15,9° (C = 2.42 Methanol); |
| | |
| 27 | (+)-2-Hydroxymethyl-5-octadecyloxy-1-(1-phenylethyl)-4(1H)-pyridon, wachsartig, IR (KBr): 1630 (C=O), [α]_{D} = +16,5° (C = 2.01 Methanol); |

### Beispiel 28

### 2-Hydroxymethyl-5-octadecyloxy-1-propyl-4(1H)-pyridon

Eine Mischung aus 20,0 g (51 mMol), 2-Hydroxymethyl-5-octadecyloxy-4-pyranon und 30 ml einer 40%igen Lösungen aus Propylamin in Ethanol wird im Autoklaven bei 100-110°C 5 Stunden erhitzt. Nach dem Abkühlen wird das gebildete Kristallisat mit Wasser aufgenommen, verrührt, abfiltriert, getrocknet und aus Ethylacetat umkristallisiert. Man erhält farblose Kristalle vom schmp. 120-121°C.

In analoger Weise, wie in Beispiel 28 beschrieben, erhält man:

| Beispiel Nr. | |
|---|---|
| 29 | 2-Hydroxymethyl-1-methyl-5-octadecyloxy-4(1H)-pyridon, Schmp. 96-99°C aus 2-Propanol; |

### Beispiel 30

### 2-Hydroxymethyl-5-octadecyloxy-1-phenyl-4(1H)-pyridon

5,0 g (12 mMol) 2-Hydroxymethyl-5-octadecyloxy-4-pyranon werden in 90 ml Wasser und 2 ml konz. Salzsäure suspendiert und mit 4,0 g (43 mMol) Anilin versetzt. Die Mischung wird 24 Stunden am Rückfluß erhitzt, abgekühlt, der sich gebildete Niederschlag von der wäßrigen Lösung abgetrennt und aus Ethylacetat umkristallisiert. Man erhält ein fast farbloses Produkt vom Schmp. 67-72°C.

In analoger Weise, wie in Beispiel 30 beschrieben, erhält man:

| Beispiel Nr. | |
|---|---|
| 31 | 1-(4-Benzyloxyphenyl)-2-hydroxymethyl-5-octadecyloxy-4(1H)-pyridon, Schmp. 68-70°C, roh weiterverarbeitet; |

### Beispiel 32

### 1-Benzyl-2-brommethyl-5-octadecyloxy-4-(1H)-pyridon, Hydrobromid

Zur Lösung von 26,5 g (0,1 Mol) Triphenylphosphin in 600 ml absol. Toluol werden unter Rühren bei Raumtemperatur 5,1 ml (o,1 Mol) Brom zugetropft, wobei sich ein weißer, kristalliner Niederschlag bildet. Nach 20 Minuten Weiterrühren bei Raumtemperatur werden 48,17 g (0,1 Mol) 1-Benzyl-2-hydroxymethyl-5-octadecyloxy-4(1H)-pyridon in Portionen zugefügt. Danach wird die Reaktionsmischung 2 Stunden bei 70°C gerührt, abgekühlt, das ausgefallene Produkt abfiltriert, mit Toluol gewaschen, getrocknet und aus 2-Propanol umkristallisiert. Man erhält hellbeige Kristalle vom Schmp. 92-95°C.

In analoger Weise, wie in Beispiel 32 beschrieben, erhält man:

| Beispiel Nr. | |
|---|---|
| 34 | 1-Benzyl-2-brommethyl-5-decyloxy-4(1H)-pyridon. Hydrobromid, Sirupartig, roh weiterverarbeitet; |
| | |
| 35 | 1-Benzyl-1-brommethyl-5-tetradecyloxy-4(1H)-pyridon. Hydrobromid, Schmp. 135-137°C, mit Aceton verrührt; |
| | |
| 36 | 1-Benzyl-2-brommethyl-5-eicosanyloxy-4(1H)-pyridon Hydrobromid, Schmp. 97-100°C, mit Aceton verrührt; |
| | |
| 37 | 1-Benzyl-2-brommethyl-5-(9-cis-octadecenyloxy)-4(1H)-pyridon . Hydrobromid; |
| | |
| 38 | 2-Brommethyl-1-(4-methylbenzyl)-5-octadecyloxy-4(1H)-pyridon . Hydrobromid, Schmp. 106-110°C aus Toluol; |
| | |
| 39 | 2-Brommethyl-1-(4-chlorbenzyl)-5-octadecyloxy-4(1H)-pyridon . Hydrobromid, Schmp. 138-148°C, mit Toluol verrührt; |
| | |
| 40 | 3-Brommethyl-1-(4-methoxybenzyl)-5-octadecyloxy-4(1H)-pyridon . Hydrobromid, schmp. 94-97°C aus Toluol; |
| 41 | 2-Brommethyl-1-(2-methoxybenzyl)-5-octadecyloxy-4(1H)-pyridon . Hydrobromid, roh weiterverarbeitet; |
| | |
| 42 | 2-Brommethyl-1-cyclohexylmethyl-5-octadecyloxy-4(1H)-pyridon . Hydrobromid, roh weiterverarbeitet; |
| | |
| 43 | 2-Brommethyl-1-(4-dimethylaminobenzyl)-5-octadecyloxy-4(1H)-pyridon . Hydrobromid, roh weiterverarbeitet; |
| | |
| 44 | 2-Brommethyl-5-octadecyloxy-1-propyl-4(1H)-pyridon . Hydrobromid, Schmp. 85-87°C aus Aceton; |
| | |
| 45 | 2-Brommethyl-1-methyl-5-octadecyloxy-4(1H)-pyridon . Hydrobromid, Schmp. 147-148°C aus Aceton; |
| | |
| 46 | 2-Brommethyl-5-octadecyloxy-1-phenyl-4(1H)-pyridon . Hydrobromid, Schmp. 136-140°C aus 2-Propanol; |
| | |
| 47 | 1-(4-Benzyloxybenzyl)-2-brommethyl-5-octadecyloxy-4(1H)-pyridon . Hydrobromid, roh weiterverarbeitet; |
| | |
| 48 | 1-(4-Benzyloxyphenyl)-2-brommethyl-5-octadecyloxy-4(1H)-pyridon . Hydrobromid, roh weiterverarbeitet; |

### Beispiel 49

### 1-Benzyl-2-brommethyl-5-(2-octyldecyloxy)-4(1H)-pyridon · Hydrobromid

Zur Lösung von 1,3 g (5 mMol) Triphenylphosphin in 10 ml Dichlormethan wird unter Rühren bei Raumtemperatur die Lösung von 0,25 ml (5 mMol) Brom in 2,5 ml Dichlormethan zugetropft. Nach weiteren 20 Minuten Rühren wird die Lösung von 2,4 g (5mMol) 2-Hydroxymethyl-5-(2-octyldecyloxy)-4-pyranon in 10 ml Dichlormethan zugetropft. Die Reaktionsmischung wird 4 Stunden unter Rückfluß gekocht. Nach dem Abkühlen vird das Lösungsmittel abdestilliert, der Rückstand mit Ether verrührt, das gebildete Kristallisat abfiltriert, getrocknet und ohne weitere Reinigung umgesetzt.

In analoger Weise, wie in Beispiel 49 beschrieben, erhält man:

| Beispiel Nr. | |
|---|---|
| 50 | 5-[2-(1-Adamantyl)ethoxy]-1-benzyl-2-brommethyl-4(1H)-pyridon · Hydrobromid, Schmp. 200-203°C aus 2-Propanol; |
| | |
| 51 | (±)-2-Brommethyl-5-octadecyloxy-1-(1-phenylethyl)-4(1H)-pyridon · Hydrobromid, roh weiterverarbeitet; |
| | |
| 52 | (-)-2-Brommethyl-5-octadecyloxy-1-(1-phenylethyl)-4(1H)-pyridon · Hydrobromid, roh weiterverarbeitet; |
| | |
| 53 | (+)-2-Brommethyl-5-octadecyloxy-1-(1-phenylethyl)-4(1H)-pyridon · Hydrobromid, roh weiterverarbeitet; |

### Beispiel 54

### 1-Benzyl-2-[N-(3-dimethylaminopropyl)aminomethyl]-5-octadecyloxy-4(1H)-pyridon . Dioxalat

Zur Lösung von 2,5 g (25 mMol) 3-Dimethylamino-1-propylamin in 30 ml 2-Propanol werden unter Rühren bei Raumtemperatur 3,1 g (5 mMol) 1-Benzyl-2-brommethyl-5-octadecyloxy-4(1H)-pyridon . Hydrobromid in Portionen eingetragen. Das erhaltene Gemisch wird 1,5 Stunden bei 60°C gerührt, abgekühlt, im Vakuum eingedampft, der Rückstand mit Wasser versetzt und mit Dichlormethan extrahiert. Die Dichlormethanlösung wird über Na₂SO₄ getrocknet, filtriert und im Vakuum zu einem ölartigen Feststoff eingedampft, dieser wird durch Säulenchromatographie über Kieselgel unter Elution mit Dichlormethan/Methanol, NH₃ ges., 9:1, gereinigt und dann in Methanol aufgelöst. Die Lösung wird mit einer Lösung von Oxalsäure in Methanol versetzt, das ausgefallene Salz abgesaugt und aus Methanol/Wasser 2,5:1 umkristallisiert. Man erhält farblose Kristalle vom Schmp. 161-164°C.

In analoger Weise, wie in Beispiel 54 beschrieben, erhält man:

| Beispiel Nr. | |
|---|---|
| 44 | 1-Benzyl-2-[N-(3-hydroxypropyl)aminomethyl]-5-octadecyloxy-4(1H)-pyridon, Schmp. 89-91°C aus Diisopropylether/2-Propanol; |
| | |
| 56 | 2-[N-(3-Dimethylaminopropyl)aminomethyl]-1-methyl-5-octadecyloxy-4(1H)-pyridon. Trihydrochlorid . 1/2 H₂O, Schmp. 230°C (Zers.) aus Ethanol; |
| | |
| 57 | 1-Benzyl-2-[N-(2-dimethylaminoethyl)-N-methylaminomethyl]-5-octadecyloxy-4(1H)-pyridon . Trihydrochlorid, Schmp. 172-174°C aus 2-Propanol; |
| | |
| 58 | 2-[N-(3-Hydroxypropyl)aminomethyl]-1-methyl-5-octadecyloxy-4(1H)-pyridon. Schmp. 79-82°C mit Ether digeriert; |
| | |
| 59 | 2-[N-(3-Dimethylaminopropyl)aminomethyl]-5-octadecyloxy-1-propyl-4(1H)-pyridon . Dioxalat, Schmp. 171-172°C aus Methanol; |
| | |
| 60 | 2-[N-(3-Aminopropyl)aminomethyl]-1-methyl-5-octadecyloxy-4(1H)-pyridon . Trihydrochlorid, Schmp. 212-215°C aus Ethanol; |
| | |
| 61 | 2-[N-(3-Aminopropyl)aminomethyl]-1-benzyl-5-octadecyloxy-4(1H)-pyridon . Trihydrochlorid, Schmp. 180-183°C aus Ethanol; |
| | |
| 62 | 2-[N-(3-Aminopropyl)aminomethyl]-1-propyl-5-octadecyloxy-4(1H)-pyridon . Trihydrochlorid, Schmp. 204-205°C aus 2-Propanol/Methanol; |
| | |
| 64 | 1-Benzyl-5-decyloxy-2-[N-(3-dimethylaminopropyl)-aminomethyl]-4(1H)-pyridon . Dioxalat . 1/2 H₂O, Schmp. 166-167°C aus 2-Propanol; |
| | |
| 65 | 1-Benzyl-2-[N-(3-dimethylaminopropyl)aminomethyl]-5-tetradecyloxy-4(1H)-pyridon . Dioxalat . 1 H₂O, Schmp. 163-167°C aus Methanol/Wasser; |
| | |
| 66 | 1-Benzyl-2-[N-(3-dimethylaminopropyl)aminomethyl]-5-eicosanyloxy-4(1H)-pyridon . Diaxalat . 1 H₂O, Schmp. 165°C aus 2-Propanol/H₂O; |
| | |
| 67 | 2-[N-(2-Aminoethyl)aminomethyl]-1-benzyl-5-octadecyloxy-4(1H)-pyridon . 1/4 H₂O, Schmp. 87-89°C aus Diisopropylether; |
| | |
| 68 | 1-Cyclohexylmethyl-2-[N-(3-dimethylaminopropyl)-aminomethyl]-5-octadecyloxy-4(1H)-pyridon . Difumarat, Schmp. 142-145°C aus Ethanol; |
| | |
| 69 | 1-(4-Chlorbenzyl)-2-[N-(3-dimethylaminopropyl)-aminomethyl)-5-octadecyloxy-4(1H)-pyridon . Difumarat ·. 1.25 H₂O, Schmp. 121-123°C aus Ethanol; |
| 70 | 2-[N-(3-Dimethylaminopropyl)aminomethyl)-5-octadecyloxy-1-phenyl-4(1H)-pyridon, Schmp. 50-52°C aus Diisopropylether; |
| | |
| 71 | 1-Benzyl-2-[N-(3-N-benzyl-N-methylaminopropyl)-aminomethyl]-5-octadecyloxy-4(1H)-pyridon . Difumarat, schmp. 140-142°C aus Ethanol; |
| | |
| 72 | 2-[N-(3-Dimethylaminopropyl)aminomethyl]-1-(4-methoxybenzyl)-5-octadecyloxy-4(1H)-pyridon . Trihydrochlorid . H₂O, Schmp. 140-145°C aus 2-Butanon/2-Propanol: |
| | |
| 73 | 1-Benzyl-2-dimethylaminomethyl-5-octadecyloxy-4(1H)-pyridon . oxalat . 1/2 H₂O, Schmp. 138-139°C aus 2-Propanol; |
| | |
| 74 | 2-[N-(3-Dimethylaminopropyl)aminomethyl]-1-(2-methoxybenzyl)-5-octadecyloxy-4(1H)-pyridon . Difufmarat, Schmp. 108-112°C aus 2-Propanol; |
| | |
| 75 | 1-Benzyl-2-(N-benzyl-N-methylaminomethyl)-5(octadecyloxy-4(1H)-pyridon, Schmp. 59-61 °C aus Ligroin; |
| | |
| 76 | 2-[N-(3-Dimethylaminopropyl)aminomethyl]-1-(4-methylbenzyl)-5-octadecyloxy-4(1H)-pyridon . Trihydrochlorid . 1/2 H₂O, Schmp. 141-148°C aus Ethylacetat/2-Propanol; |
| | |
| 77 | 2-[N-(4-Aminobutyl)aminomethyl]-1-benzyl-5-octadecyloxy-4(1H)-pyridon . Trihydrochlorid, Schmp. 179-183°C aus Ether/Ethylacetat: |
| | |
| 78 | 1-Benzyl-2-[N-(3-dimethylaminopropyl)aminomethyl]-5-(9-cis-octadecenyloxy)-4(1H)-pyridon · Trihydrochlorid · 1/2 H₂O, Schmp. 181-185°C aus Ethylacetat; |
| 79 | 1-(4-Benzyloxyphenyl)-2-[N-(3-dimethylaminopropyl)-aminomethyl]-5-octadecyloxy)-4(1H)-pyridon, Schmp. 43-47°C; |
| | |
| 80 | 1-(4-Benzyloxybenzyl)-2-[N-(3-dimethylaminopropyl)-aminomethyl]-5-octadecyloxy)-4(1H)-pyridon · Difumarat · 2,5 H₂O, Schmp. 103-109°C aus 2-Propanol/Ethylacetat; |
| | |
| 81 | 1-Benzyl-z-[N-(3-dimethylaminopropyl)aminomethyl]-5-(2-octyldecyloxy)-4(1H)-pyridon · Trihydrochlorid · 3/4 H₂O, Schmp. 168-172°C aus Methylethylketon/2-Propanol; |
| | |
| 82 | 5-(2-Adamantylethoxy)-1-benzyl-2-[N-(3-dimethylaminopropyl)aminomethyl]-4(1H)-pyridon, gelbes Öl, MS (m/l) 477 M⁺; |
| | |
| 83 | (±)-1-Benzyl-5-octadocyloxy)-2-(N-[3-(1-phenylethyl)aminopropyl]aminomethyl)-4(1H)-pyridon · Trihydrochlorid, Schmp. 125-127°C mit Toluol verrührt: |

### Beispiel 84

### 2-[N-(3-Dimethylaminopropyl)aminomethyl]-1-(4-hydroxybenzyl)-5-octadecyloxy-4(1H)-pyridon · Dihydrochlorid · 2 H₂O

Eine Lösung von 1,0 g (1,5 mMol) 1-(4-Benzyloxybenzyl)-2-[N-(3-dimethylaminopropyl)aminomethyl]-5-octadecyloxy-4(1H)-pyridon in 30 ml absolutem Ethanol wird mit 0,25 g Palladium auf Kohle (10%ig, Oxidform) versetzt und bei Raumtemperatur und Normaldruck hydriert. Nach Filtration vom Katalysator wird das Lösungsmittel abdestilliert und der Rückstand an Kieselgel mit Dichlormethan Mit 3-6% Methanol mit NH₃-Gas gesättigt säulenchromatographiert. Das so erhaltene Reaktionsprodukt (wachsartige Substanz mit dem R_{f} = 0,55, Kieselgel, Dichlormethan/Methanol mit NH₃ gesättigt 4:1) wird mit HCl-Gasgesättigtem Methanol zur Salzbildung versetzt. Das Lösungsmittel wird abdestilliert und der Rückstand aus 2-Propanol/Ethylacetat umkristallisiert. Man erhält farblose Kristalle vom Schmp. 153-156°C.

In analoger Weise, wie in Beispiel 84 beschrieben, erhält man:

| Beispiel Nr. | |
|---|---|
| 85 | 2-(N-(3-Dimethylaminopropyl)aminomethyl)-1-(4-hydroxyphenyl)-5-occadecyloxy-4(1H)-pyridon · Trihydrochlorid · 0,5 H₂O, Schmp. 236-237°C aus Methanol; |

### Beispiel 86

### 1-[(1-Benzyl-5-octadecyloxy-4(1H)-pyridon-2-yl)methyl]-4-cyano-4-phenylpiperidin . Dihydrochlorid

Eine Lösung von 2,0 g (9 mMol) 4-Cyano-4-phenylpiperidin · Hydrochlorid in 40 ml Ethanol wird mit 4,8 ml (35 mMol) Triethylamin versetzt. Zu diesem Gemisch worden unter Rühren bei Raumtemperatur 5,0 g (8 mMol) 1-Benzyl-2-brommethyl-5-octadecyloxy-4(1H)-pyridon · Hydrobromid in Portionen eingetragen. Die Mischung wird 3 Stunden bei 60°C gerührt, das Lösungsmittel im vakuum abgezogen, der Rückstand mit Wasser und Dichlormethan versetzt, verrührt, die organische Phase abgetrennt, über Na₂SO₄ getrocknet und eingedampft. Der Rückstand wird zur Reinigung an Kieselgel mit Dichlormethan/Methanol 97:3 säulenchromatographiert. Man erhält ein öliges Produkt. 1,0 g (1,5 mMol) Substanz werden in 30 ml Ethylacetat gelöst und mit HCl-Gas-gesättigten Ether zur Salzbildung versetzt. Das ausgefallene Salz wird aus Ethylacetat/2-Propanol 6:1 umkristellisiert. Man erhält farblose Kristalle vom Schmp. 106-112°C, wobei der Feststoff etwa 3/4 Mol H₂O pro Mol des genannten Produktes enthält.

In analoger Weise, wie in Beispiel 86 beschrieben, erhält man:

| Beispiel Nr. | |
|---|---|
| 87 | 1-[(1-Benzyl-5-octadecyloxy-4(1H)-pyridon-2-yl)methyl]-4-methyloxycarbonyl-2-methylpiperazin . Dihydrochlorid · 3/4 H₂O, Schmp. 111-117°C aus Ethylacetat/2-Propanol; |
| | |
| 88 | 1-[(1-Benzyl-5-octadecyloxy-4(1H)-pyridon-2-yl)methyl]-4-benzylpiperazin, Schmp. 70-80°C aus Diisopropylether; |
| | |
| 89 | 1-Benzyl-2-[N-(3-dimethylaminopropyl)-N-methylaminomethyl]-5-octadecyloxy-4(1H)-pyridon · Trihydrochlorid, Schmp. 186-189°C aus 2-Propanol/Ethanol ; |
| | |
| 90 | 1-[(1-Benzyl-5-octadecyloxy-4(1H)-pyridon-2-yl)-methyl]-4-diphenylmethylpiperazin · 1,5 oxalat · H₂O, Schmp. 124-129°C aus Ethylacetat; |
| | |
| 91 | 1-[(1-Benzyl-5-octadecyloxy-4(1H)-pyridon-2-yl)-methyl]-4-phenylpiperazin · Dihydrochlorid · H₂O, Schmp. 80°C (sintern) aus Ethylacetat/Methanol; |
| | |
| 92 | 1-[(1-Benzyl-5-octadecyloxy-4(1H)-pyridon-2-yl)methyl)-4-(2-pyrimidinyl)piperazin, Schmp. 96-97°C aus Diisopropylether/2-Propanol; |
| | |
| 93 | 1-[(1-Benzyl-5-octadecyloxy-4(1H)-pyridon-2-yl)methyl]-4-benzylpiperazin · Dioxalat · 1/2 H₂O, Schmp. 133-136°C aus Ethanol; |
| | |
| 94 | 1-[(1-Benzyl-5-octadecyloxy-4(1H)-pyridon-2-yl)-methyl]-4-(2-phenylethyl)piperazin · Trihydrochlorid · 1/2 H₂O, Schmp. 161-164°C aus Ethylacetat/2-Propanol; |
| | |
| 95 | 1-((1-Benzyl-5-decyloxy-4(1H)-pyridon-2-yl)methyl]-4-(2-phenylethyl)piperazin · Dihydrochlorid, Schmp. 161-164°C aus Ethylacetat/2-Propanol; |
| | |
| 96 | 1-[(1-Benzyl-5-benzyloxy-4(1H)-pyridon-2-yl)methyl)-4-benzylpiperazin · Trihydrochlorid, Schmp. 189-191°C aus Ethylacetat/2-Propanol; |
| | |
| 97 | (±)-1-[(1-Benzyl-5-octadecyloxy-4(1H)-pyridon-2-yl)methyl]-4-(1-phenylethyl)piperazin · Trihydrochlorid 3/4 H₂O, Schmp. 167-170°C aus Acetonitril/2-Propanol; |
| | |
| 98 | (±) -1-[(5-Octadecyloxy-1-(1-phenylethyl)-4(1H)-pyridon-2-yl)methyl]-4-benzylpiperazin, Schmp. 76-78°C aus Diisopropylether; |
| | |
| 99 | (-) -1-[(5-Octadecyloxy-1-(1-phenylethyl)-4(1H)-pyridon-2-yl)methyl]-4-benzylpiperazin, Schmp. 60-61°C aus Diisopropylether, [α]_{D} = -2,3° (C = 2,06 Methanol) ; |
| | |
| 100 | (+)-1-[(5-octadecyloxy-1-(1-phenylethyl)-4(1H)-pyridon-2-yl)methyl]-4-benzylpiparazin, Schmp. 60-61°C aus Diisopropylether, [α]_{D} = +2,4° (C = 2,01 Methanol); |

### Beispiel 101

### 1-[(1-Benzyl-5-octadecyloxy-4(1H)-pyridon-2-yl)methyl]-4-aminomethyl-4-phenylpiperidin · Trihydrochlorid

2,7 g (4,1 mMol) 1-[(1-Benzyl-5-octadecyloxy-4 (1H)-pyridon-2-yl)methyl]-4-cyano-4-phenylpiperidin werden mit 60 ml NH₃-Gasgesättigtem Methanol und 1 g Raney Nickel versetzt und bei 60°C und 60 bar im Autoklaven mit Wasserstoff hydriert. Nach 6 Stunden ist die Reaktion beendet. Nach Abfiltration des Katalysators wird die hellgelbe Lösung im Vakuum eingedampft und der Rückstand an Xieselgel mit Dichlormethan/Methanol, mit NH₃ gesättigt, 97:3, säulenchromatographiert. Das so erhaltene Reaktionsprodukt (ölige Substanz mit dem Rf = 0,5; Kieselgel, Dichlormethan/ Methanol mit NH₃ gesätt., 9:1) wird in Ethylacetat gelöst und mit HCl-Gas-gesättigtem Ether zur Salzbildung versetzt. Das ausgefallene Salz wird aus 2-Butanon/2-Propanol 10:1 umkristallisiert. Man erhält farblose Kristalle vom Schmp. 192-197°C. Der Feststoff enthält etwa 1,25 Mol Wasser pro Mol des genannten Produktes.

### Beispiel 102

### (+/-)-1-[(1-Benzyl-5-octadecyloxy-4(1H)-pyridon-2-yl)methyl]-2-methylpiperazin · Fumarat

Eine Mischung aus 2 g (3,2 mMol) (+/-)-1-[(1-Benzyl-5-octadecyloxy-4(1H)-pyridon-2-yl) methyl]-4-methoxycarbonyl-2-methylpiperazin, 5 ml 33% HBr/Essigsäure und 5 ml Essigsäure wird 2 Stunden im geschlossenen Gefäß gerührt. Nach Abziehen der Hauptmenge des Lösungsmittel im Vakuum wird der Rückstand mit Wasser versetzt, mit 2N Natronlauge alkalisiert, mit Dichlormethan extrahiert, die organische Phase abgetrennt, über Na₂SO₄ getrocknet und eingedampft. Das erhaltene Harz wird zur Reinigung an Kieselgel mit Dichlormethan/Methanol, mit NH₃-Gas gesättigt, 97:3 säulenchromatographiert. Das dabei erhaltene Reaktionsprodukt (ölige Substanz mit dem Rf = 0,2; Kieselgel, Dichlormethan/Methanol mit NH₃-Gas-gesättigt 9:1) wird in 2-Propanol gelöst und mit einer Lösung von Fumarsäure in 2-Propanol versetzt. Nach 24 Stunden Stehen wird die klare lösung eingedampft und das Salz aus 2-Butanon umkristallisiert. Man erhält farblose Kristalle vom Schmp. 151-155°C. Der Feststoff enthält etwa 1 Mol H₂O pro Mol des genannten Produktes.

### Beispiel 103

### 1-Benzyl-2-(3-dimethylaminopropyloxymethyl)-5-octadecyloxy-4(1H)-pyridon · Dihydrochlorid

7,2 g (15 mMol) 1-Benzyl-2-hydroxymethyl-5-octadecyloxy-4(1H)-pyridon werden in 110 ml absol. Dimethylformamid bei 70°C gelöst und portionsweise mit 0,60 g (20 mMol) Natriumhydrid (80% in Paraffinöl) versetzt. Nach 1,5 Stunden Nachrühren bei 70-80°C wird eine Lösung von 3,3 g (27,15 mMol) 3-Dimethylaminopropylchlorid in 20 ml absol. Dimethylformamid zugetropft. Die Reaktionsmischung läßt man 2 Stunden bei 70°C rühren. Danach dampft man die Lösung im Vakuum zur Trockene ein und versetzt den Rückatand mit Wasser und Dichlormethan. Die organische Phase wird abgetrennt, über Na₂SO₄ getrocknet und eingedampft. Der Rückstand wird an Kieselgel mit Dichlormethan/ Methanol mit NH₃-Gas gesättigt 97:3 säulenchromatographiert. Man erhält eine ölige Substanz. 1,8 g (3,3 mMol) Substanz werden in ein Gemisch aus 30 ml Ethylacetat und 3,5 ml 2-Propanol gelöst und mit HCl-Gas gesättigten Ether zur Salzbildung versetzt. Das ausgefallene Salz wird aus Ethylacetat/ 2-Propanol 3:1 umkristallisiert. Man erhält farblose Kristalle vom Schmp. 155-159°C. Der Feststoff enthält etwa 1/4 Mol Wasser pro Mol des genannten Produktes.

In analoger Weise, wie in Beispiel 103 beschrieben, erhält man:

| Beispiel Nr. | |
|---|---|
| 104 | 2-(3-Dimethylaminopropyloxymethyl)-1-methyl-5-octadecyloxy-4(1H)-pyridon · Dihydrochlorid, Schmp. 210-216°C aus 2-Propanol; |
| | |
| 105 | 2-(3-Dimethylaminopropyloxymethyl)-5-octadecyloxy-1-propyl-4(1H)-pyridon · Dihydrochlorid . 3/4 H₂O, Schmp. 127-128°C aus Ethylacetat/2-Propanol; |
| | |
| 106 | 2-(3-Dimethylaminopropyloxymethyl)-1-(4-methylbenzyl)-5-octadecyloxy-4 pyridon · Dihydrochlorid · 1,5 H₂O, Schmp. 78-84°C aus Ethylacetat/2-Propanol; |
| | |
| 107 | 2-(3-Dimethylaminopropyloxymethyl)-1-(4-methoxybenzyl)-5-octadecyloxy-4(1H)-pyridon . Dihydrochlorid · 1/4 H₂O, Schmp. 144-147°C aus 2-Propanol; |
| | |
| 108 | 1-Cyclohexylmethyl-2-(3-dimethylaminopropyloxymethyl)-5-octadecyloxy-4(1H)-pyridon · Oxalat, Schmp. 81-84°C aus Ethylacetat/2-Propanol; |
| | |
| 109 | 1-(4-Chlorbenzyl)-2-(3-dimethylaminopropyloxymethyl)-5-octadecyloxy-4(1H)-pyridon · Dihydrochlorid · 2,5 H₂O, Schmp. 70-74°C aus Ethylacetat/2-Propanol) ; |
| | |
| 110 | 2-(3-Dimethylaminopropyloxymethyl)-1-(2-methoxybenzyl)-5-octadecyloxy-4(1H)-pyridon · 1,5 oxalat, Schmp. 89-91°C aus Ethylacetat/2-Propanol; |

### Beispiel 111

### 3-[1-Benzyl-5-octadecyloxy-4(1H)-pyridon-2-yl]-propionsäurenitril . Hydrochlorid

### A. 3-[1-Benzyl-5-octadecyloxy-4(1H)-pyridon-2-yl]-2-cyanpropionsäure-tert.-butylester

Zur Lösung von 17,0 g (0,15 Mol) Kalium-tert.-butylat in 60 ml absol. Dimethylformamid wird unter Rühren bei Raumtemperatur die Lösung von 21,2 g (0,15 Mol) Cyanessigsäure-tert.-butylester in 15 ml absol. Dimethylformamid getropft. Die klare Lösung wird auf 50°C erwärmt und bei dieser Temperatur eine Lösung von 43,4 g (0,07 Mol) l-Benzyl-2-brommethyl-5-octadecyloxy-4(1H)-pyridon . Hydrobromid in 150 ml absol. Dimethylformamid in 5 Minuten zugegeben. Nach 1 Stunde Rühren bei 55°C wird das Reaktionsgemisch zu 1,2 l Wasser gegeben und mit Ethylacetat extrahiert. Die organische Phase wird abgetrennt, über Na₂SO₄ getrocknet und eingeengt. Der ölige Rückstand wird zur Reinigung an Kieselgel mit Dichlormethan/Methanol 99:1 säulenchromatographiert (Rf = 0,60; Kieselgel, Dichlormethan/Methanol 9:1). Man erhält ein gelbes, öliges Produkt.

### B. 3-[1-Benzyl-5-octadecyloxy-4 (1H)-pyridon-2-yl]-2-cyanpropionsäure

13,2 g (22 mMol) 3-[1-Benzyl-5-octadecyloxy-4(1H)-pyridon-2-yl]-2-cyan-propionsäure-tert.-butylester werden mit 47 ml 0,5 N Kaliumhydroxid in Methanol versetzt und 3 Tage bei Raumtemperatur gerührt. Das Lösungsmittel wird im Vakuum abgezogen, der Rückstand in Wesser gelöst und mit 2 N Salzsäure angesäuert, der ausgefallene Niederschlag abgesaugt, mit Wasser und Ether gewaschen und getrocknet. Man erhält ein fast farbloses Produkt vom Schmp. 122-124°C, das für die weitere Umsetzung genügend rein ist.

### C. 3-[1-Benzyl-5-octadecyloxy-4(1H)-pyridon-2-yl]-propionsäurenitril . Hydrochlorid

4.1 g (7,4 mMol) 3-[1-Benzyl-5-octadecyloxy-4(1H)-pyridon-2-yl]-2-cyan-propionsäure werden 30 Minuten bei 150°C erhitzt. Nach dem Abkühlen erhält man das Produkt als Öl, das für die weitere Umsetzung genügend rein ist.

0,5 g (1 mMol) Substanz werden in Ethylacetat gelöst und mit HCl-Gas gesättigtem Ether zur Salzbildung versetzt. Das ausgefallene Salz vird abgesaugt und getrocknet. Man erhält ein beiges Produkt vom Schmp. 98-102°C.

In analoger weise, wie in Beispiel 111 beschrieben, erhält man:

| Beispiel Nr.: | |
|---|---|
| 112 | 3-(1-Methyl-5-octadecyloxy-4(1H)-pyridon-2-yl)-propionsaurenitril, Schmp. 85-87°C mit Ether digeriert ; |

### Beispiel 113

### 3-[1-Benzyl-5-octadecyloxy-4(1H)-pyridon-2-yl]propylamin · Dihydrochlorid

3,7 g (7,3 mMol) 3-[1-Benzyl-5-octadecyloxy-4(1H)-pyridon-2-yl)-propionsäurenitril werden mit 40 ml mit NH₃-Gas-gesättigtem Methanol versetzt und bei 60°C und 60 bar im Autoklaven mit Wasserstoff hydriert. Nach Filtration vom Katalysator wird die Lösung im Vakuum eingedampft und der ölige Rückstand an Kieselgel mit Dichlormethan/Methanol, mit NH₃ gesättigt, 95:5, säulenchromatographiert. So erhält man 2,2 g (59 % d. Th.) ölige Substanz (Rf = 0,1; Kieselgel, Dichlormethan/Methanol mit NH₃ gesättigt 9:1), die mit etherischer Salzsäure in das Hydrochlorid übergeführt und aus 2-Butanon/2-Propanol 4:1 umkristallisiert wird. Man erhält farblose Kristalle vom Schmp. 138-143°C.

### Beispiel 114

### 3-[1-Benzyl-5-octadecyloxy-4(1H)-pyridon-2-yl]propionsäuremethylester

3,0 g (6 mMol) 3-[1-Benzyl-5-octadecyloxy-4(1H)-pyridon-2-yl]-propionsäurenitril werden in 30 ml mit HCl-Gas-gesättigtem Methanol gelöst und 3 Tage bei Raumtemperatur im verschlossenen Gefäß stehen gelassen. Nach Abziehen des Lösungsmittels im Vakuum wird der Rückstand mit Wasser versetzt, mit verd. Natriumhydroxidlösung alkalisiert und mit Dichlormethan extrahiert. Die Dichlormethanlösung wird über Na₂SO₄ getrocknet und eingedampft. Das erhaltene Wachs wird zweimal aus Diisopropylether umkristallisiert. Man erhält farblose Kristalle vom Schmp. 78-81°C.

### Beispiel 115 und 116

### 3-[1-Benzyl-5-octadecyloxy-4(1H)-pyridon-2-yl]propanol und 3-[1-Benzyl-5-octadecyloxy-4(1H)-pyridon-2-yl]propionsäureamid

5,0 g (10 mMol) 3-[1-Benzyl-5-Octadecyloxy-4(1H)-pyridon-2-yl]-propionsäurenitril in 30 ml 2-Propanol werden unter Rühren bei Raumtemperatur mit 2,5 g (6,6 mMol) Natriumborhydrid versetzt und das Gemisch 4 Tage bei 80°C weitergeührt. Nach Abziehen des Lösungsmittels im Vakuum wird der Rückstand mit verd. Natriumhydroxidlösung versetzt und 30 Minuten bei 40°C gerührt. Nach Abkühlen wird mit Dichlormethan extrahiert, die organische Phase mit Wasser gewaschen, getrocknet und eingedampft. Der Rückstand wird an Kieselgel mit Dichlormethan/Methanol säulenchromatographiert. Durch Elution mit Dichlormethan mit 1-5 % Methanol wird der rohe 3-[1-Benzyl-5-octadecyloxy-4(1H)-pyridon-2-yl]propanol erhalten (Rf = 0,30; Kieselgel, Dichlormethan/Methanol 9:1). Nach Umkristallisation aus Diisopropylether/ 2-Propanol 4:1 erhält man hellbeige Kristalle vom Schmp. 87-89°C.
Durch weitere Elution der Säule mit Dichlormethan mit 6-10% Methanol wird das rohe 3-[1-Benzyl-5-octadecyloxy-4(1H)-pyridon-2-yl]-propionsäureamid erhalten (Rf = 0,40; Kieselgel, Dichlormethan/Methanol 9:1). Nach Umkristallisation aus 2-Butanon erhält man farblose Kristalle vom Schmp. 134-136°C.

### Hemmung der Aktivität von Proteinkinase C

Zur Klärung des hemmenden Einflusses der erfindungsgemäßen Verbindungen der allgemeinen Formel I auf die Calcium- und Phospholipid-abhängige Proteinkinase (PKC) wurde diese Enzymaktivität aus Rattenhirn angereichert. In Anlehnung an in der Literatur beschriebene Reinigungsschritte (J. Biol. Chem. 260, 15718-15722, 1985) wurde bei der aus 2 Schritten bestehenden Reinigung die Eigenschaft des Enzyms ausgenutzt, in Anwesenheit von calcium an Zellmembranen zu binden und durch calciumchelatoren (EGTA) wieder abgelöst zu verden. Beim 1. Anreicherungsschritt erfolgte die Bindung der PKC an Membranen des Rattenhirns und im 2. Schritt an sogenannte Inside/OutVesikel von Erythrozyten. Nach Ablösung von den Erythrozyten-Membranen und Umpufferung lag die PKC-Präparation in 10mM HEPES, 1 mM DTT, 0,1% PEG 20000, pH 7,5 vor und konnte unter diesen Bedingungen bei -70°C für mehrere Monate ohne Aktivitätsverlust gelagert verden.

Die Aktivität des Enzyms wurde über den Einbau von phosphor-32-markiertem Phosphat in das durch PKC phosphorylierbare Protein Histon H-1 (Sigma Typ III) bestimmt.

Der Test enthält dabei folgende Komponenten:

50 mM HEPES-NaOH (pH 7,5), 5 mM MgCl₂, 1 mM DTT, 4 µm freies Ca², 10 µM ATP, 1 µg Phosphatidylserin, 0,2 µg 1,2-Diolein sowie 40 µg Histon H-1 und gegebenenfalls die Testsubstanz.

Der Ansatz wird 4 min bei 30°C vorinkubiert und die Reaktion dann durch Zusatz von 5 nM PXC gestartet. Nach 5 min Inkubation bei 30°C vird die Reaktion mit 10% TCA gestoppt und die Proben dann abfiltriert. Der Phosphateinbau wird mittels CerenkovZählung im Scintillationszähler bestimmt. Die in Abwesenheit der Testsubstanz gemesasene Kinase-Aktivität wurde jeweils als 100% gesetzt und die Hemmwirkung der Verbindungen der allgemeinen Formel I prozentual darauf bezogen (Tabelle I).

### Endothelabhängige Relaxation der Gefäßmuskulatur

Zur Anwendung kommen Gefäßringe der Kaninchenaorta, die zwischen zwei L-förmigen Haken im Organbad fixiert werden. Die Präparate befinden sich in einer physiologischen Salzlösung (nach Krebs-Henseleit), die von Carbogen durchperlt wird. Die Kontraktionen werden durch 3 x 10⁻⁷ mol/l Noradrenalin ausgelöst. Wenn die Spannung ein stabiles Plateau erreicht hat, erfolgt die Zugabe der Testsubstanzen. Die substanzwirkung wird zuerst in Präparaten mit intaktem Endothel und anschließend, um die Endothelabhängigkeit der gefundenen Effekte zu überprüfen, in Präparaten mit zerstörtem Endothel getestet. Für eine endothelabhängige Relaxation, die durch den vom Endothel stammenden relaxierenden Faktor (EDRF, endothelium-derived relaxing factor) bewirkt wird, gelten die Kriterien: Abwesenheit des Effektes in Präparaten mit zerstörtem Endothel, keine Hemmbarkeit der Relaxation durch Indometazin und Hemmbarkeit durch NDGA (Nordihydro-guaiaretsäure), Methylenblau und Gossypol. Diese Kriterien werden von den in der Tabelle II angeführten Verbindungen voll erfüllt, so daß gefolgert werden kann, daß die angeführten Verbindungen unerwarteterweise über die Freisetzung des EDRF virken.

**Tabelle I**

| Beispiel | Hemmung der Proteinkinase C |
|---|---|
| Nr. | (IC₅₀; Mol/ml) |
| 19 | 2,4 x 10⁻⁶ |
| 54 | 1,4 x 10⁻⁶ |
| 56 | 2,9 x 10⁻⁶ |
| 57 | 1,9 x 10⁻⁶ |
| 59 | 2,9 x 10⁻⁶ |
| 60 | 2,7 x 10⁻⁶ |
| 61 | 2,1 x 10⁻⁶ |
| 65 | 6,5 x 10⁻⁶ |
| 66 | 2,5 x 10⁻⁶ |
| 68 | 1,9 x 10⁻⁶ |
| 69 | 1,5 x 10⁻⁶ |
| 70 | 2,2 x 10⁻⁶ |
| 71 | 1,8 x 10⁻⁶ |
| 72 | 3,6 x 10⁻⁶ |
| 73 | 4,4 x 10⁻⁶ |
| 74 | 2,1 x 10⁻⁶ |
| 75 | 9,5 x 10⁻⁶ |
| 76 | 1,9 x 10⁻⁶ |
| 77 | 2,3 x 10⁻⁶ |
| 78 | 4,6 x 10⁻⁶ |
| 79 | 2,2 x 10⁻⁶ |
| 80 | 2,6 x 10⁻⁶ |
| 83 | 2,6 x 10⁻⁶ |
| 84 | 3,4 x 10⁻⁶ |
| 85 | 5,8 x 10⁻⁶ |
| 88 | 3,0 x 10⁻⁶ |
| 89 | 1,8 x 10⁻⁶ |
| 93 | 5,5 x 10⁻⁶ |
| 94 | 2,4 x 10⁻⁶ |
| 95 | 4,3 x 10⁻⁶ |
| 97 | 4,4 x 10⁻⁶ |
| 98 | 5,4 x 10⁻⁶ |
| 99 | 5,2 x 10⁻⁶ |
| 100 | 2,7 x 10⁻⁶ |
| 101 | 1,5 x 10⁻⁶ |
| 102 | 2,0 x 10⁻⁶ |
| 103 | 4,0 x 10⁻⁶ |
| 105 | 2,0 x 10⁻⁶ |
| 106 | 2,4 x 10⁻⁶ |
| 107 | 2,9 x 10⁻⁶ |
| 108 | 2,9 x 10⁻⁶ |
| 109 | 2,6 x 10⁻⁶ |
| 114 | 9,0 x 10⁻⁶ |
| 116 | 9,0 x 10⁻⁶ |

**Tabelle II**

| Beispiel | Relaxation der Noradrenalin (3 x 10⁻⁷ mol/l) vorkontrahierten Aorta: EC₅₀, mol/l | |
|---|---|---|
| Nr. | Endothel intakt | Endothel entfernt |
| 54 | 1,5 x 10⁻⁵ | > 10⁻⁴ |
| 101 | 2 x 10⁻⁵ | > 10⁻⁴ |
| 69 | 3 x 10⁻⁵ | > 10⁻⁴ |
| 71 | 3 x 10⁻⁵ | > 10⁻⁴ |
| 88 | 3,4 x 10⁻⁵ | > 10⁻⁴ |
| Acetylcholin | 3 x 10⁻⁸ | > 10⁻⁴ |

## Patentansprüche

1. Alkoxy-4(1H)-pyridon-Derivate der allgemeinen Formel I 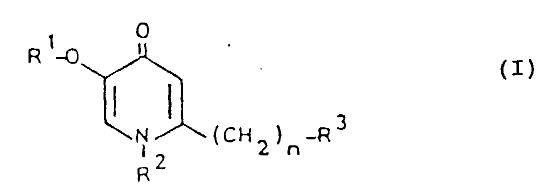 in welcher
R¹ eine geradkettige oder verzweigte, gesättigte oder ungesättigte Alkyl- oder Adamantylalkylgruppe mit jeweils bis zu 22 Kohlenstoffatomen darstellt,
R² eine geradkettige oder verzweigte, gesättigte oder ungesättigte Alkylgruppe mit bis zu 5 Kohlenstoffatomen, eine Cycloalkylmethylgruppe mit 5 bis 7 C-Atomen im Cycloalkylring, eine unsubstituierte oder durch Halogen, Hydroxy, Alkyl mit bis zu 5 C-Atomen, Alkoxy mit bis zu 5 C-Atomen, Dialkylamino mit bis zu 5 C-Atomen oder Benzyloxy substituierte Phenyl- oder Phenylalkylgruppe mit einem geradkettigen oder verzweigten Alkylrest mit bis zu 5 C-Atomen oder eine Aminoalkylgruppe der allgemeinen Formel II darstellt 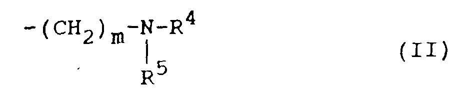 worin R⁴ und R⁵ gleich oder verschieden sind und Wasserstoff oder eine Alkylgruppe mit 1 bis 3 C-Atomen und m eine ganze Zahl von 2 bis 5 bedeuten,
n eine ganze Zahl von 1 bis 6 bedeutet, und
R³ entweder
a) ein Halogenatom, eine Hydroxy-, cyano-, Carboxamid- oder Alkoxycarbonylgruppe mit bis zu 5 C-Atomen in der Alkylgruppe bedeutet, oder einen Aminorest der allgemeinen Formel III 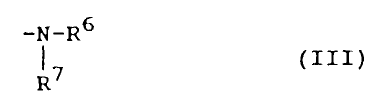 bedeutet, worin R⁶ und R⁷ gleich oder verschieden sind und Wasserstoff, eine Alkyl- oder omega-Hydroxyalkylgruppe mit bis zu 5 C-Atomen, eine unsubstituierte oder durch Halogen, Hydroxy, Alkyl mit bis zu 5 C-Atomen, Alkoxy mit bis zu 5 C-Atomen, Dialkylamino mit bis zu 5 C-Atomen oder Benzyloxy substituierte Phenyl- oder Phenylalkylgruppe mit einem geradkettigen oder verzweigten Alkylrest mit bis zu 5 C-Atomen bedeuten, oder
b) einen Aminorest der allgemeinen Formel IV 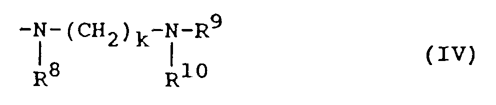 darstellt, in welcher R⁸ Wasserstoff oder eine Alkylgruppe mit bis zu 5 C-Atomen und R⁹ und R¹⁰ gleich oder verschieden sind und Wasserstoff, eine Alkylgruppe mit bis zu 5 C-Atomen, eine unsubstituierte oder durch Halogen, Hydroxy, Alkyl mit bis zu 5 C-Atomen, Alkoxy mit bis zu 5 C-Atomen, Dialkylamino mit bis zu 5 C-Atomen oder Benzyloxy substituierte Phenyl- oder Phenylalkylgruppe mit einem geradkettigen oder verzweigten Alkylrest mit bis zu 5 C-Atomen bedeuten und k eine ganze Zahl von 2 bis 5 bedeutet, oder
c) einen Heterocyclus der allgemeinen Formel V 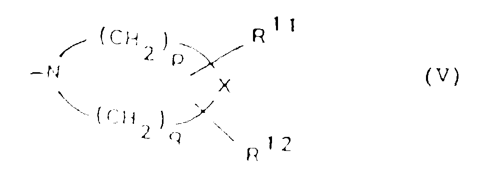 bedeutet, in welcher R¹¹ und R¹² gleich oder verschieden sind und Wasserstoff, eine Alkylgruppe mit bis zu 3 C-Atomen oder eine Phenyl- oder Phenylalkylgruppe mit einem geradkettigen oder verzweigten Alkylrest mit bis zu 5 C-Atomen bedeuten, X für Sauerstoff, einen Aminorest der allgemeinen Formel VI, 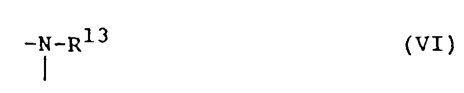 wobei R¹³ Wasserstoff oder eine Phenyl-, Phenylalkyl- oder Diphenylalkylgruppe mit einem geradkettigen oder verzweigten Alkylrest mit bis zu 5 C-Atomen, wobei im Phenylring bis zu 3 CH-Gruppen durch Stickstoffatome ersetzt sein können, bedeutet, oder für einen Rest der allgemeinen Formel VII 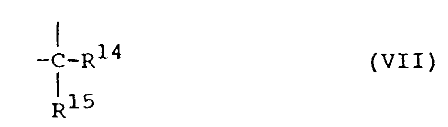 steht, worin R¹⁴ Wasserstoff oder eine unsubstituierte oder durch Halogen, Hydroxy, Alkyl mit bis zu 5 C-Atomen, Alkoxy mit bis zu 5 C-Atomen, Dialkylamino mit bis zu 5 C-Atomen oder Benzyloxy substituierte Phenylgruppe bedeutet, R¹⁵ Wasserstoff, eine Hydroxy-, Hydroxymethyl-, Cyano-, Aminomethyl-, Carboxamid-, Ethoxy- oder Methoxycarbonylgruppe und p und q gleich oder verschieden sein können und 2 oder 3 bedeuten, oder
d) für einen Rest der allgemeinen Formel VIII 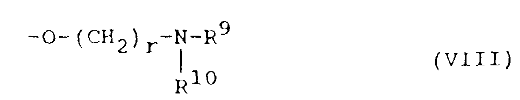 steht, worin R⁹ und R¹⁰ die oben genannte Beudeutung haben, und r eine ganze Zahl von 2 bis 5 bedeutet, sowie deren pharmakologisch unbedenkliche Salze, mit Ausnahme derjenigen Verbindungen, bei denen R¹ einen Alkylrest von 1-6 Kohlenstoffatomen bedeutet.

2. Verbindungen der allgemeinen Formel I gemäß Anspruch 1, dadurch gekennzeichnet, daß
R¹ eine geradkettige oder verzweigte, gesättigte oder ungesättigte Alkyl- oder Adamantylalkylgruppe mit jeweils bis zu 22 Kohlenstoffatomen darstellt,
R² einen geradkettigen oder verzweigten Alkylrest mit 1 bis 4 C-Atomen, einen Cyclohexylmethylrest, einen unsubstituierten oder durch Halogen, Hydroxy, Methyl, Benzyloxy, Methoxy oder Dimethylamino monosubstituierten Phenyl- oder Phenylalkylgruppe mit einem geradkettigen oder verzweigten Alkylrest mit bis zu 5 C-Atomen bedeutet,
n eine ganze Zahl von 1 bis 3 bedeutet, und
R³ entweder,
a) ein Halogenatom, eine Hydroxy-, Cyano-, Carboxamid- oder Alkoxycarbonylgruppe mit bis zu 5 C-Atomen in der Alkylgruppe oder einen Rest der allgemeinen Formel III darstellt, in welcher R⁶ und R⁷ gleich oder verschieden sind und Wasserstoff, eine Methyl-, omega-Hydroxypropyl- oder Benzylgruppe bedeuten, oder
b) einen Aminorest der allgemeinen Formel IV darstellt, in welcher R⁸ Wasserstoff oder eine Methylgruppe bedeutet, R⁹ und R¹⁰ gleich oder verschieden sind und Wasserstoff, eine Methyl-, Benzyl- oder Phenylethylgruppe und k, 2, 3 oder 4 bedeuten, oder
c) einen Heterocyclus der allgemeinen Formel V darstellt, in welcher R¹¹ und R¹² gleich oder verschieden sind und Wasserstoff oder eine Methylgruppe bedeuten, X für einen Aminorest der allgemeinen Formel VI steht, wobei R¹³ Wasserstoff oder eine Phenyl-, Phenylalkyl- oder Diphenylalkylqruppe mit einem geradkettigen oder verzweigten Alkylrest mit bis zu 5 C-Atomen, wobei im Phenylring bis zu 3 CH-Gruppen durch Stickstoffatome ersetzt sein können, bedeutet, oder X für einen Rest der allgemeinen Formel VII steht, worin R¹⁴ für eine Phenylgruppe, R¹⁵ eine Cyano- oder Aminomethylgruppe bedeutet, und p und q gleich 2 sind oder
d) einen Rest der allgemeinen Fromel VIII darstellt, in welcher R⁹ und R¹⁰ Methylgruppen bedeuten und r gleich 2 oder 3 ist, mit Ausnahme derjenigen Verbindungen, bei denen R¹ einen Alkylrest von 1-6 Kohlenstoffatomen bedeutet.

3. Verbindungen der allgemeinen Formel I gemäß Anspruch 1 oder 2, dadurch gekennzeichnet, daß
n gleich 1, 2 oder 3 ist,
R¹ einen Decyl-, Tetradecyl-, Eicosanyl-, Octadecyl-, Octadecenyl-, 2-Octyldecyl- oder Adamantylethylrest,
R² einen Propyl-, Methyl-, Phenyl-, Benzyl-, Methylbenzyl, Chlorbenzyl-, Methoxybenzyl-, Cyclohexylmethyl-, Dimethylaminobenzyl-, Dimethylaminopropyl-, Hydroxybenzyl-, Benzyloxybenzyl-, Benzyloxyphenyl-, Hydroxyphenyl- oder Phenylethylrest und
R³ einen Hydroxy-, Brom-, Cyano-, Amino-, Carboxamid-, Methoxycarbonyl-, Dimethylaminopropylamino-, Dimethylaminopropyl-N-methylamino-, Hydroxypropylamino-, Dimethylaminoethyl-N-methylamino-, Aminopropylamino-, Aminoethylamino-, Aminobutylamino-, Dimethylamino-, Dimethylaminopropyloxy-, N-Benzyl-N-methylaminopropylamino-, N-Benzyl-N-methylamino-, Methyloxycarbonyl-methylpiperazin-, Benzylpiperazin-, Diphenylmethylpiperazin-, Aminomethylphenylpiperidin-, Phenylpiperazin-, Methylpiperazin-, Phenylethylpiperazin- oder Pyrimidinylpiperazinrest bedeutet.

4. Verfahren zur Herstellung von Alkoxy-4(1H)-pyridon-Derivaten der allgemeinen Formel I gemäß Anspruch 1 bis 3, dadurch gekennzeichnet, daß man entweder
a) eine Verbindung der allgemeinen Formel IX 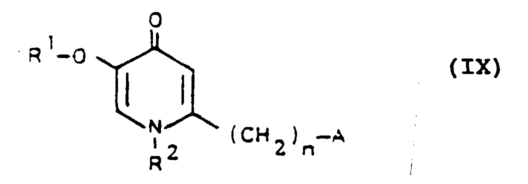 in welcher R¹, R² die oben angegebene Bedeutung haben, n gleich 1 ist und A für eine nukleofuge Abgangsgruppe steht, mit Aminoverbindungen der allgemeinen Formel X, XI und XII, 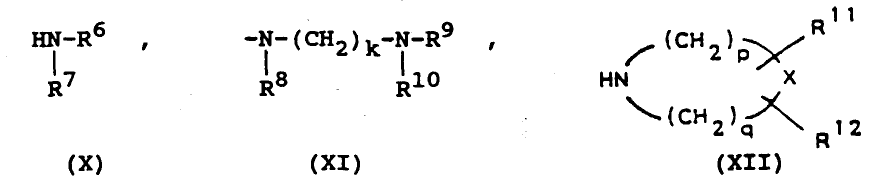 in welcher R⁶, R⁷, R⁸, R⁹, R¹⁰, R¹¹, R¹², k, p, q und X die oben angegebene Bedeutung besitzen, in an sich bekannter Weise umsetzt, oder
b) eine Verbindung der allgemeinen Formel XIII, 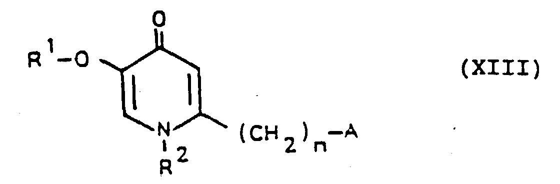 in welcher R¹, R² die oben angegebene Bedeutung haben und n gleich 1 ist, mit einer Verbindung der allgemeinen Formel XIV, 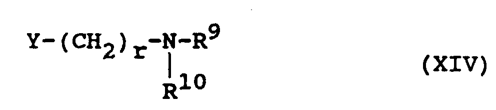 in welcher R⁹, R¹⁰ und r die oben genannte Bedeutung haben und Y eine Abgangsgruppe bedeutet, in einem geeigneten Lösungsmittel unter basischen Bedingungen, umsetzt, oder
c) wenn n gleich 2 oder 3 ist und R³ für eine Amino-, Hydroxy-, Cyano-, Methoxycarbonyl- oder Carboxamidgruppe steht, ein 2-Halogenmethylpyridon der allgemeinen Formel IXa 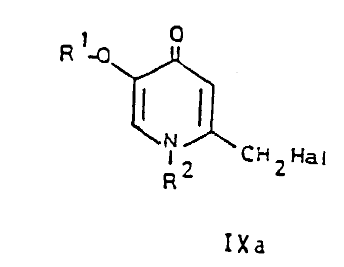 in welcher R¹ und R² die oben angegebene Bedeutung haben, mit einem Cyanessigsäureester nach an sich bekannter Weise umsetzt, verseift und decarboxyliert und aus den gemäß a), b) oder c) erhaltenen Cyanoverbindungen der allgemeinen Formel Ia 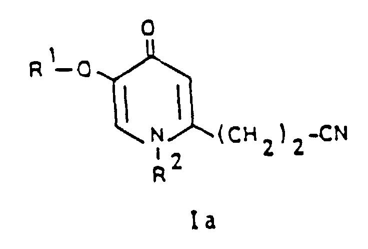 nach literaturbekannten Methoden die Ester-, Amino-, Hydroxy- und Amidoverbindungen der allgemeinen Formel I herstellt.

5. Arzneimittel enthaltend neben üblichen Hilfs- und Zusatzstoffen Verbindungen der allgemeinen Formel I gemäß Anspruch 1.

6. Verbindungen der allgemeinen Formel I gemäß Anspruch 1 zur Prävention und/oder Behandlung von Herz- und Gefäßkrankheiten wie Thrombosen, Arteriosklerose, Hypertonien, von Entzündungsprozessen, Allergien, Krebs und bestimmten degenerativen Schäden des Zentralnervensystems.

7. Verwendung von Verbindungen der allgemeinen Formel I gemäß Anspruch 1 zur Herstellung von Pharmazeutika zur Prävention und/oder Behandlung von Herz- und Gefäßkrankheiten wie Thrombosen, Arteriosklerose, Hypertonien, von Entzündungsprozessen, Allergien, Krebs und bestimmten degenerativen Schäden des Zentralnervensystems.

## Claims

1. Alkoxy-4(1H)-pyridone derivatives of the general formula I 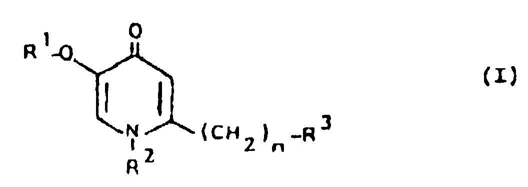 in which
R¹ represents a straight-chain or branched, saturated or unsaturated alkyl or adamantylalkyl group having in each case up to 22 carbon atoms,
R² represents a straight-chain or branched, saturated or unsaturated alkyl group having up to 5 carbon atoms, a cycloalkylmethyl group having 5 to 7 C atoms in the cycloalkyl ring, a phenyl or phenylalkyl group having a straight-chain or branched alkyl radical having up to 5 C atoms and being unsubstituted or substituted by halogen, hydroxy, alkyl having up to 5 C atoms, alkoxy having up to 5 C atoms, dialkylamino having up to 5 C atoms or benzyloxy, or an aminoalkyl group of the general formula II 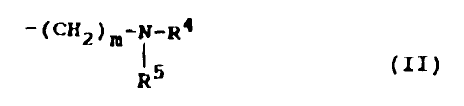
wherein R⁴ and R⁵ are the same or different and denote hydrogen or an alkyl group having 1 to 3 C atoms and m denotes a whole number from 2 to 5,
n denotes a whole number from 1 to 5, and
R³ denotes either
a) a halogen atom, a hydroxy, cyano, carboxamide or alkoxycarbonyl group having up to 5 C atoms in the alkyl group, or denotes an amino radical of the general formula III 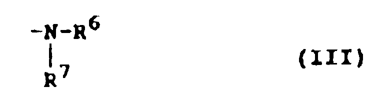 wherein R⁶ and R⁷ are the same or different, and denote hydrogen, an alkyl or omega-hydroxyalkyl group having up to 5 C atoms, a phenyl or phenylalkyl group having a straight-chain or branched alkyl radical having up to 5 C atoms and being unsubstituted or substituted by halogen, hydroxy, alkyl having up to 5 C atoms, alkoxy having up to 5 C atoms, dialkylamino having up to 5 C atoms or benzyloxy, or represents
b) an amino radical of the general formula IV 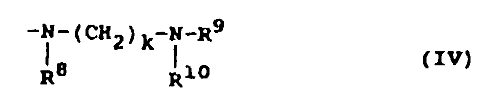 in which R⁸ denotes hydrogen or an alkyl group having up to 5 C atoms and R⁹ and R¹⁰ are the same or different and denote hydrogen, an alkyl group having up to 5 C atoms, a phenyl or phenylalkyl group having a straight-chain or branched alkyl radical having up to 5 C atoms and being unsubstituted or substituted by halogen, hydroxy, alkyl having up to 5 C atoms, alkoxy having up to 5 C atoms, dialkylamino having up to 5 C atoms or benzyloxy, and k denotes a whole number from 2 to 5, or denotes
c) a heterocycle of the general formula V 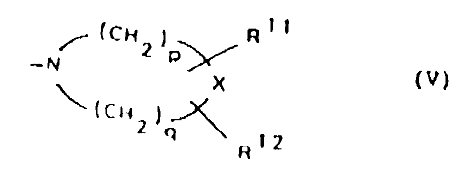 in which R¹¹ and R¹² are the same or different and denote hydrogen, an alkyl group having up to 3 C atoms or a phenyl or phenylalkyl group having a straight-chain or branched alkyl radical having up to 5 C atoms, X represents oxygen, an amino radical of the general formula VI, 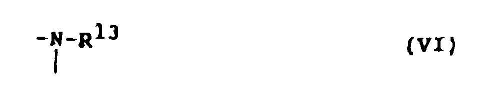 wherein R¹³ denotes hydrogen or a phenyl, phenylalkyl or diphenylalkyl group having a straight-chain or branched alkyl radical having up to 5 C atoms, wherein in the phenyl ring up to 3 CH groups may be replaced by nitrogen atoms, or represents a radical of the general formula VII 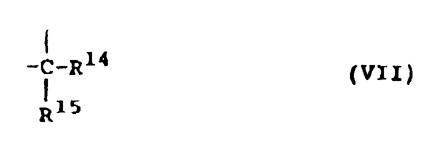 wherein R¹⁴ denotes hydrogen or a phenyl group unsubstituted or substituted by halogen, hydroxy, alkyl having up to 5 C atoms, alkoxy having up to 5 C atoms, dialkylamino having up to 5 C atoms or benzyloxy, R¹⁵ denotes hydrogen, a hydroxy, hydroxymethyl, cyano, aminomethyl, carboxamide, ethoxy or methoxycarbonyl group, and p and q may be the same or different and denote 2 or 3, or represents
d) a radical of the general formula VIII 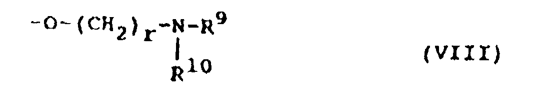 wherein R⁹ and R¹⁰ have the above-mentioned meaning, and r denotes a whole number from 2 to 5, as well as their pharmacologically acceptable salts, with the exception of those compounds in which R¹ denotes an alkyl radical of 1-6 carbon atoms.

2. Compounds of the general formula I according to claim 1, characterised in that
R' represents a straight-chain or branched, saturated or unsaturated alkyl or adamantylalkyl group having in each case up to 22 carbon atoms,
R² denotes a straight-chain or branched alkyl radical having 1 to 4 C atoms, a cyclohexylmethyl radical, a phenyl or phenylalkyl group having a straight-chain or branched alkyl radical having up to 5 C atoms and being unsubstituted or monosubstituted by halogen, hydroxy, methyl, benzyloxy, methoxy or dimethylamino,
n denotes a whole number from 1 to 3, and
R³ represents either
a) a halogen atom, a hydroxy, cyano, carboxamide or alkoxycarbonyl group having up to 5 C atoms in the alkyl group or a radical of the general formula III, in which R⁶ and R⁷ are the same or different and denote hydrogen, a methyl, omega-hydroxypropyl or benzyl group, or represents
b) an amino radical of the general formula IV in which R⁸ denotes hydrogen or a methyl group, R⁹ and R¹⁰ are the same or different and denote hydrogen, a methyl, benzyl or phenylethyl group and k denotes 2, 3 or 4, or represents
c) a heterocycle of the general formula V, in which R¹¹ and R¹² are the same or different and denote hydrogen or a methyl group, X represents an amino radical of the general formula VI, wherein R¹³ denotes hydrogen or a phenyl, phenylalkyl or diphenylalkyl group having a straight-chain or branched alkyl radical having up to 5 C atoms, wherein in the phenyl ring up to 3 CH groups may be replaced by nitrogen atoms, or X represents a radical of the general formula VII, wherein R¹⁴ represents a phenyl group, R¹⁵ denotes a cyano or aminomethyl group, and p and q are equal to 2, or represents
d) a radical of the general formula VIII, in which R⁹ and R¹⁰ denote methyl groups and r is equal to 2 or 3, with the exception of those compounds in which R¹ denotes an alkyl radical of 1-6 carbon atoms.

3. Compounds of the general formula I according to claim 1 or 2, characterised in that
n is equal to 1, 2 or 3,
R¹ denotes a decyl, tetradecyl, eicosanyl, octadecyl, octadecenyl, 2-octyldecyl or adamantylethyl radical,
R² denotes a propyl, methyl, phenyl, benzyl, methylbenzyl, chlorobenzyl, methoxybenzyl, cyclohexylmethyl, dimethylaminobenzyl, dimethylaminopropyl, hydroxybenzyl, benzyloxybenzyl, benzyloxyphenyl, hydroxyphenyl or phenylethyl radical, and
R³ denotes a hydroxy, bromo, cyano, amino, carboxamide, methoxycarbonyl, dimethylaminopropylamino, dimethylaminopropyl-N-methylamino, hydroxypropylamino, dimethylaminoethyl-N-methylamino, aminopropylamino, aminoethylamino, aminobutylamino, dimethylamino, dimethylaminopropyloxy, N-benzyl-N-methylaminopropylamino, N-benzyl-N-methylamino, methyloxycarbonyl-methylpiperazine, benzylpiperazine, diphenylmethylpiperazine, aminomethylphenyipiperidine, phenylpiperazine, methylpiperazine, phenylethylpiperazine or pyrimidinylpiperazine radical.

4. Process for producing alkoxy-4(1H) -pyridone derivatives of the general formula I according to claim 1 to 3, characterised in that either
a) a compound of the general formula IX 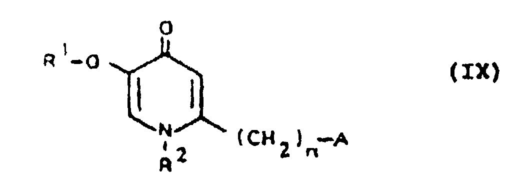 in which R¹, R² have the meaning given above, n is equal to 1 arid A represents a nucleophobic leaving group, is reacted with amino compounds of the general formula X, XI and XII, 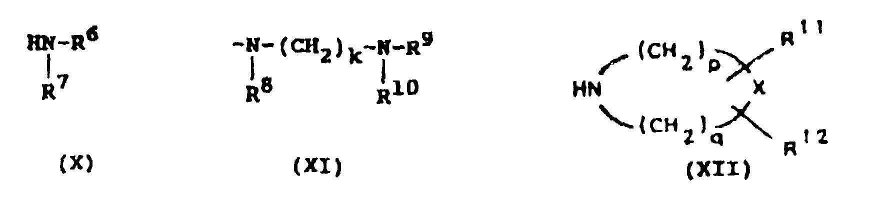 in which R⁶, R⁷, R⁸, R⁹, R¹⁰, R¹¹, R¹², k, p, q and X have the meaning given above, in a manner known per se, or
b) a compound of the general formula XIII, 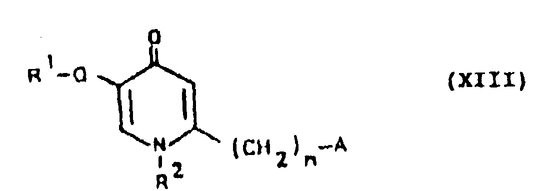 in which R¹, R² have the meaning given above and n is equal to 1, is reacted with a compound of the general formula XIV, 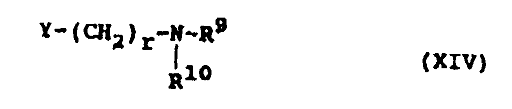 in which R⁹, R¹⁰ and r have the above-mentioned meaning and Y denotes a leaving group, in a suitable solvent under alkaline conditions, or
c) when n is equal to 2 or 3 and R³ represents an amino, hydroxy, cyano, methoxycarbonyl or carboxamide group, a 2-halomethylpyridone of the general formula IXa 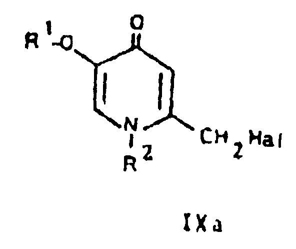 in which R¹ and R² have the meaning given above, is reacted with a cyanoacetate according to a manner known per se, saponified and decarboxylated, and the ester, amino, hydroxy and amido compounds of the general formula I are produced from the cyano compounds of the general formula Ia 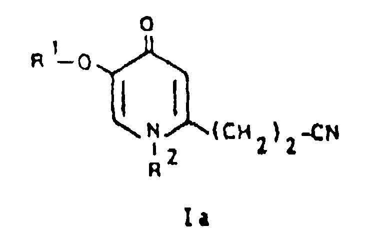 obtained according to a), b) or c) by methods known in the literature.

5. Medicaments containing compounds of the general formula I according to claim 1 in addition to conventional auxiliaries and additives.

6. Compounds of the general formula I according to claim 1 for prevention and/or treatment of cardiac and vascular diseases, such as thromboses, arteriosclerosis, hypertension, of inflammatory processes, allergies, cancer and certain degenerative damage to the central nervous system.

7. Use of compounds of the general formula I according to claim 1 for producing pharmaceuticals for prevention and/or treatment of cardiac and vascular diseases, such as thromboses, arteriosclerosis, hypertension, of inflammatory processes, allergies, cancer and certain degenerative damage to the central nervous system.

## Revendications

1. Composés alkoxy-4(1H)-pyridon de formule générale I 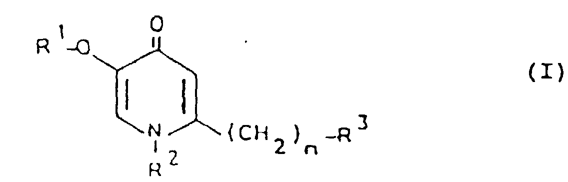 dans laquelle
R¹ est un groupe alkyle ou adamantylalkyle à chaîne droite ou ramifiée, saturé ou insaturé, ayant chaque fois jusqu'à 22 atomes de carbone,
R² est un groupe alkyle à chaîne droite ou ramifiée, saturé ou insaturé, ayant jusqu'à 5 atomes de carbone, un groupe cycloalkylméthyle ayant de 5 à 7 atomes de C dans le cycle cycloalkyle, un groupe phényle ou phénylalkyle non substitué ou substitué par halogène. hydroxy, alkyle ayant jusqu'à 5 atomes de C, alkoxy ayant jusqu'à 5 atomes de C, dialkylamino ayant jusqu'à 5 atomes de C ou benzyloxy, ayant un résidu alkyle à chaîne droite ou ramifié ayant jusqu'à 5 atomes de C ou un groupe aminoalkyle de formule générale II : 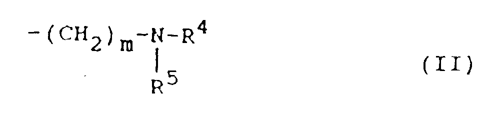 dans laquelle R⁴ et R⁵ sont identiques ou différents et sont l'hydrogène ou un groupe alkyle ayant de I à 3 atomes de C et m est un nombre entier de 2 à 5,
n est un nombre entier de 1 à 5, et
R³ soit,
a) est un atome d'halogéné, un groupe hydroxy, cyano, carboxamide ou alkoxycarbonyle ayant jusqu'à 5 atomes de C dans le groupe alkyle. ou un résidu amino de formule générale III 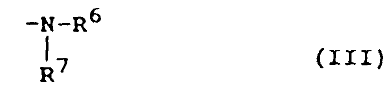 dans laquelle R⁶ et R⁷ sont identiques ou différents et sont l'hydrogène, un groupe alkyle ou oméga-hydroxyalkyle ayant jusqu'à 5 atomes de C, un groupe phényle ou phénylalkyle non substitué ou substitué par halogène, hydroxy alkyle avec jusqu'à 5 atomes de C, alkoxy ayant jusqu'à 5 atomes de C, dialkylamino ayant jusqu'à 5 atomes de C ou benzyloxy, avec un résidu alkyle à chaîne droite ou ramifié ayant jusqu'à 5 atomes de C, ou
b) un résidu amino de formule générale IV 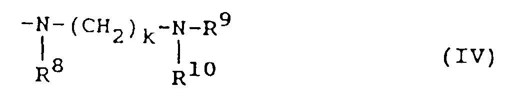 dans laquelle R⁸ est l'hydrogène ou un groupe alkyle ayant jusqu'à 5 atomes de C, et R⁹ et R¹⁰ sont identiques ou différents et sont l'hydrogène, un groupe alkyle ayant jusqu'à 5 atomes de C, un groupe phényle ou phénylalkyle non substitué ou substitué par halogène, hydroxy, alkyle ayant jusqu'à 5 atomes de C, alkoxy ayant jusqu'à 5 atomes de C, dialkylamino ayant jusqu'à 5 atomes de C ou benzyloxy, avec un résidu alkyle à chaîne droite ou ramifié, ayant jusqu'à 5 atomes de C, et k est un nombre entier de 2 à 5, ou
c) un hétérocycle de formule générale V 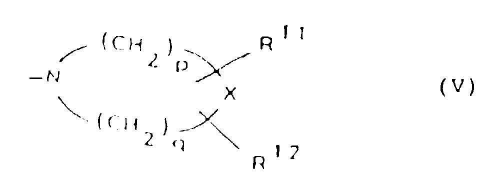 dans laquelle R¹¹ et R¹² sont identiques ou différents et sont l'hydrogène, un groupe alkyle ayant jusqu'à 3 atomes de C, ou un groupe phényle ou phénylalkyle avec un résidu alkyle à chaîne droite ou ramifié ayant jusqu'à 5 atomes de C. x étant l'oxygène, un résidu amino de formule générale VI 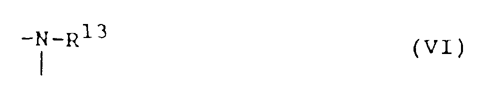 dans laquelle R¹³ est l'hydrogène ou un groupe phényle, phénylalkyle ou diphénylalkyle avec un résidu alkyle à chaîne droite ou ramifié ayant jusqu'à 5 atomes de C, ou dans le cycle phényle. jusqu'à 3 groupes CH pouvant être remplacés par des atomes d'azote, ou désigne un résidu de la formule générale VII 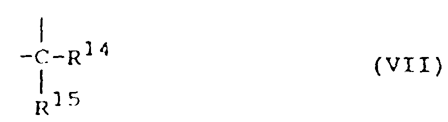 dans laquelle R¹⁴ est l'hydrogène ou un groupe phényle non substitué ou substitué par halogène. hydroxy, alkyle ayant jusqu'à 5 atomes de C, alkoxy ayant jusqu'à 5 atomes de C, dialkylamino ayant jusqu'à 5 atomes de C ou benzyloxy, R¹⁵ est l'hydrogène, un groupe hydroxy, hydroxyméthyle, cyano, aminométhyle, carboxamide, éthoxy ou méthoxycarbonyle, et p et q pouvant être identiques ou différents et valoir 2 ou 3, ou
d) désigne un résidu de formule générale (VIII) 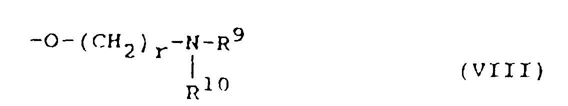 dans laquelle R9 et R10 ont la signification indiquée ci-dessus et r est un nombre entier de 2 à 5, ainsi que leurs sels pharmacologiquement acceptables, à l'exception des combinaisons pour lesquelles R¹ est un résidu alkyle ayant de 1-6 atomes de carbone.

2. Composés de formule générale I selon la revendication 1, caractérisés en ce que
R¹ est un groupe alkylc ou adamantylalkyle à chaîne droite ou ramifié, saturé ou insaturé, ayant chaque fois jusqu'à 22 atomes de carbone,
R² est un résidu alkyle à chaîne droite ou ramifiée, ayant 1 à 4 atomes de C, un résidu cyclohexylméthyle, un groupe phényle ou phénylalkyle non substitué ou mono-substitué par halogène, hydroxy, méthyle, benzyloxy, méthoxy ou diméthyalmino, avec un résidu alkyle à chaîne droite ou ramifié ayant jusqu'à 5 atomes de C,
n est un nombre entier de 1 à 3, et
R³ est soit,
a) un atome d'halogène, un groupe hydroxy, cyano, carboxamide ou alkoxycarbonyle ayant jusqu'à 5 atomes de C dans le groupe alkyle, ou un résidu de formule générale III, dans laquelle R⁶ et R⁷ sont identiques ou différents et sont l'hydrogène, un groupe méthyle, oméga-hydroxypropyle ou benzyle, ou
b) un résidu amino de formule générale IV, dans laquelle R⁸ est l'hydrogène ou un groupe méthyle, R⁹ et R¹⁰ sont identiques ou différents et sont l'hydrogène, un groupe méthyle, benzyle ou phényléthyle, et k valant 2, 3 ou 4, ou
c) un hétérocycle de formule générale V, dans laquelle R¹¹ et R¹² sont identiques ou différents et sont l'hydrogène ou un groupe méthyle, X est un résidu amino de formule générale VI, R¹³ étant l'hydrogène ou un groupe phényle, phénylalkyle ou diphénylalkyle avec un résidu alkyle à chaîne droite ou ramifié ayant jusqu'à 5 atomes de C, dans le cycle phényle jusqu'à 3 groupes CH pouvant être remplacés par des atomes d'azote, ou X étant un résidu de formule générale VII, R¹⁴ étant un groupe phényle, R¹⁵ un groupe cyano ou aminométhyle, et p et q étant égal à 2, ou
(d) un résidu de formule générale (VIII), dans laquelle R⁹ et R¹⁰ sont des groupes méthyles et r étant égal à 2 ou 3, à l'exception des combinaisons pour lesquelles R¹ est un résidu alkyle ayant de 1 à 6 atomes de carbone.

3. Composés de formule générale I selon la revendication 1 ou 2, caractérisés en ce que
n est égal à 1 , 2 ou 3,
R¹ est un résidu décyle, tétradécyle, eicosanyle, octadécyle, octadécényle, 2-octyldécyl- ou adamantyléthyle,
R² est un résidu propyle, méthyle, phényle, benzyle. méthylbenzyle, chlorobenzyle, méthoxybenzyle, cyclo-hexylméthyle, dimetenylamino-benzyle, diméthylaminopropyle, hydroxybenzyle, benzyloxybenzyle, benzyloxyphényle, hydroxyphényle ou phényléthyle, et
R³ est un résidu hydroxy, brome, cyano carboxamide, méthoxycarbonyle, diméthylaminopropylamino, diméthyl-aminopropyl-N-méthylamino, hydroxypropylamino, diméthyl-aminoéthyl-N-méthylamino, aminopropylamino, aminoéthyl-amino, aminobutylamino, diméthylamino, diméthylaminopropyloxy, N-benzyl-N-méthylaminopropyl- amino, N-benzyl-N-méthylamino, méthyloxycarbonyl- méthyl-pipérazine, benzylpipérazine, diphénylméthyl- pipérazine, aminométhylphénylpiperidine phényl- pipérazine, méthylpipérazine, phényléthylpipérazine, ou pyrimidinylpipérazine.

4. Fabrication de dérivés alkoxy-4(1H)-pyridon de formule générale I selon les revendications 1 à 3, caractérisée en ce que, soit
a) on convertit de manière connue en soi un composé de formule générale IX, 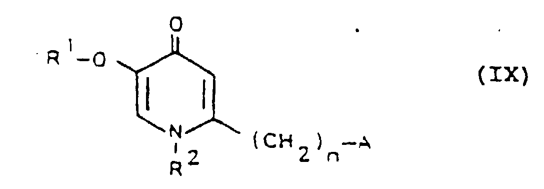 dans laquelle R¹, R² ont la signification indiquée ci-dessus, n étant égal à 1 et A étant un groupe dérivé nucléofuge, avec des composés amino de formule générale X, XI et XII, 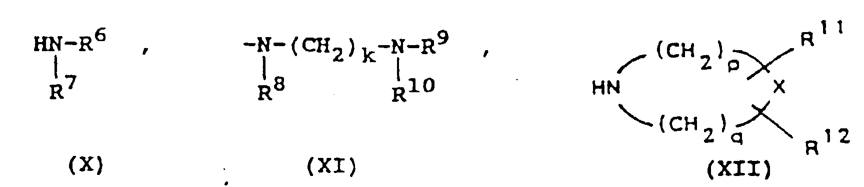 dans lesquelles R⁶, R⁷, R⁸, R⁹, R¹⁰, R¹¹, R¹², k, p, q et X ont la signification indiquée ci-dessus, soit
b) on convertit un composé de formule générale XIII, 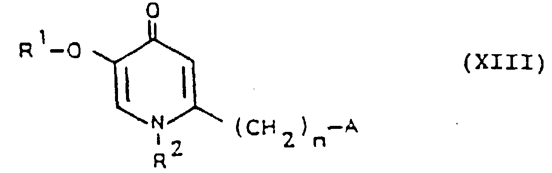 dans laquelle R¹, R² ont la signification indiquée ci-dessus et n étant égal à 1, avec un composé de formule générale XIV, 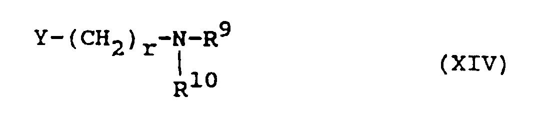 dans laquelle R⁹, R¹⁰ et r ont la signification citée ci-dessus et Y étant un groupe pendant, dans un solvant approprié, dans des conditions basiques, ou
c) lorsque n est égal à 2 ou 3 et R³ étant un groupe amino, hydroxy, cyano, méthoxycarbonyle ou carboxamide, on convertit, on saponifie et on décarboxyle un 2-halogèneméthylpyridon de formule générale IXa, 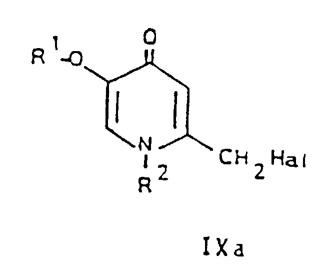 dans laquelle R¹ et R² ont la signification indiquée ci-dessus, par un ester cyanacétique, de manière connue en soi, et, à partir des composés cyano obtenus suivant a), b) ou c), de formule générale la 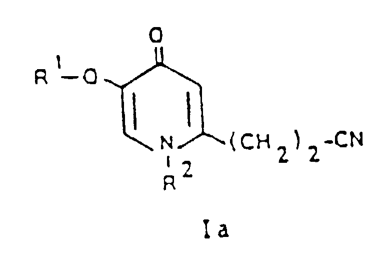 d'après les méthodes connues dans la littérature, on fabrique les composés ester, amino, hydroxy et amido de formule générale I.

5. Médicament contenant, outre les auxiliaires ou adjuvants usuels, des composés de formule générale I selon la revendication 1.

6. Composés de formule générale I selon la revendication 1, pour la prévention et/ou le traitement des maladies cardiaques et vasculaires telles que les thromboses, artérioscléroses, hypertension et processus inflammatoires, allergies, cancers et certains désordres dégénératifs du système nerveux central.

7. Utilisation des composés de formule générale I selon la revendication 1, pour la fabrication de produits pharmaceutiques pour la prévention et/ou le traitement des maladies cardiaques et vasculaires, telles que les thromboses, l'artériosclérose. l'hypertension. les processus inflammatoires, les allergies, le cancer et certains désordres dégénératifs du système nerveux central.
